# EUROPEAN PATENT SPECIFICATION

(11) **EP 3 076 951 B1**
(45) Date of publication and mention of the grant of the patent: **30.09.2020**
(21) Application number: 14808594.7
(22) Date of filing: 03.12.2014
(51) Int. Cl.: A61K 9/16, A61K 9/51, A61K 31/445, A61K 31/40, A61K 31/505, A61K 31/554, A61K 31/381, A61K 31/496, A61K 31/407, A61K 31/4168, A61K 31/403, A61K 31/4184, A61K 31/428

(54) **PROCESS FOR THE PRODUCTION OF DRUG FORMULATIONS FOR ORAL ADMINISTRATION**
VERFAHREN ZUR HERSTELLUNG VON ARZNEIMITTELFORMULIERUNGEN ZUR ORALEN VERABREICHUNG
PROCÉDÉ POUR LA PRODUCTION DE FORMULATIONS DE MÉDICAMENT POUR ADMINISTRATION ORALE

(30) Priority: 05.12.2013 EP 13195835
(43) Date of publication of application: 12.10.2016
(73) Proprietor: Albayrak, Celal, 10117 Berlin (DE)
(72) Inventor: ALBAYRAK, Celal, 13125 Berlin (DE)
(74) Representative: Seuss, Thomas
(86) International application number: PCT/EP2014/076455
(87) International publication number: WO 2015/082562

(56) References cited:
- EP-A1- 1 344 520
- WO-A1-03/020245
- WO-A1-2004/096259
- WO-A1-2009/109844
- WO-A2-02/49619
- WO-A2-2007/023495
- WO-A2-2008/075320
- US-A- 5 102 668
- US-A- 5 650 173
- US-A- 5 792 477
- US-A1- 2013 197 127
- NORDSTIERNA L ET AL: "Molecular release from painted surfaces: Free and encapsulated biocides", PROGRESS IN ORGANIC COATINGS, ELSEVIER BV, NL, vol. 69, no. 1, 1 September 2010 (2010-09-01), pages 45-48, XP027122751, ISSN: 0300-9440 [retrieved on 2010-07-06]
- DATABASE WPI Section Ch, Week 201311 Thomson Scientific, London, GB; Class A96, AN 2012-M75503 XP002734497, SHU D; XIONG S; YIN X: "Sustained-release microsphere comprises felodipine, ethyl cellulose, and drug release regulating agent, where the microsphere is prepared by emulsification method", -& CN 102 579 362 A ((UYZT) UNIV ZHEJIANG TECHNOLOGY) 18 July 2012 (2012-07-18)
- DATABASE WPI Section Ch, Week 200377 Thomson Scientific, London, GB; Class A96, AN 2003-817239 XP002723700, HANASHIMA N; MARUO S: "Sustained release external composition e.g. lotion or ointment, comprises medicine and base material having plasticity dispersed in non porous polymer microparticle", -& JP 2003 089631 A ((TEIS-N) TEISAN SEIYAKU KK) 28 March 2003 (2003-03-28)
- SAHANA D K ET AL: "PLGA NANOPARTICLES FOR ORAL DELIVERY OF HYDROPHOBIC DRUGS: INFLUENCE OF ORGANIC SOLVENT ON NANOPARTICLE FORMATION AND RELEASE BEHAVIOR IN VITRO AND IN VIVO USING ESTRADIOL AS A MODEL DRUG", JOURNAL OF PHARMACEUTICAL SCIENCES, vol. 97, no. 4, April 2008 (2008-04), pages 1530-1542, XP002723702,
- DATABASE WPI Section Ch, Week 201276 Thomson Scientific, London, GB; Class A96, AN 2012-M75499 XP002723701, FU H; PAN X; WEN X; WU C; ZHOU Y: "Sustained release microsphere, prepared using pharmaceutical active ingredient e.g. succinate and metoprolol hydrochloride, and sustained-release material containing mixture of ethyl cellulose and poly(amino-methacrylate)", -& CN 102 579 368 A (UNIV SUN YET-SEN; GUANGZHOU WANZE PHARM TECHNOLOGY CO LTD) 18 July 2012 (2012-07-18)

## Description

This invention is directed to a process for the production of drug loaded polymeric nano- and/or micro particles suitable for oral administration, and to particle formulations obtainable by the process.

Oral delivery remains the most favourable and preferred route of drug administration. Currently, more than 60 % of drugs are formulated and marketed as oral products (Oral Controlled Release Formulation Design and Drug Delivery Hong Wen, Kinam Park (Edts.) John Wiley & Sons, 2010; Formulating Poorly Water Soluble Drugs, Robert O. Williams, Alan B. Watts, Dave A. Miller (Edts.), Springer 2012). However, the drug faces several challenges after oral administration before the site of action is reached. Many drugs cannot be effectively delivered via an oral route in their original or active form due to reasons of instability, poor solubility and active efflux transport mechanisms. In spite of these issues, the oral route of drug administration represents the most preferred means of drug delivery to systemic circulation in the body. Overcoming the problems associated with oral administration is currently one of the challenging goals in drug delivery.

There are many drugs on the market or undergoing clinical trials, especially drugs belonging to class II or class III according to the BCS or BDDCS classification system, which exhibit low bioavailability. The bioavailability of these drugs is frequently restricted either by a low general solubility, or a pronounced first- pass effect. They may further be affected by the fact that they are unstable in gastrointestinal tract (GIT), or may not be targeted to the right place in the gastrointestinal tract. In addition, low bioavailability causes high inter-and intra-subject variability, that makes the prediction of the pharmacologic and toxic effects of given dose extremely difficult, especially for drugs with very narrow therapeutic indices.

Several strategies have been employed to improve the bioavailability of drugs after oral administration.

For example, targeting of the colon is highly desirable for the systemic delivery of protein and peptide drugs, and/or the local treatment of a variety of bowel diseases such as ulcerative colitis, Crohn's disease, amebiosis, and colonic cancer. However, the colon provides significant challenges to drug delivery. The colon is about 1.5 m long, with a surface area of about 0.33 m². The main factor hindering drug distribution and dissolution in the colon is the lack of fluid, resulting in a generally viscous environment. Targeting of the colon via the oral route can be achieved by different approaches including different formulation systems, for which the drug is released controlled by different pH or by the colonic physiology and local environment e.g. activity of colonic bacteria. Drugs reaching the colon should thus be already in dissolved form, either present in solid solution or in amorphous phase.

Formulations which aim at increasing the bioavailability of poorly soluble drugs, or of drugs exhibiting a pronounced first-pass effect, or which intend to reduce inter-individual variations are described in the literature. For example, US 2011/0059175, discloses a strategy for increasing the solubility of poorly water soluble drugs which uses polyvinyl alcohol-polyethylene glycol graft copolymers (PVA-PEG graft-co-polymer), such as Kollicoat IR, to produce solid dispersions formulations of low soluble drugs. Increasing solubility of sparingly soluble drugs by spray drying methods using hydroxypropyl methylcellulose acetate succinate (HPMC AS) are described in US 8623128 B2, US 8257741 B2, or US 837118 B2. US 6028054 discloses a method for the increasing of bioavailability of an oral formulation via incorporation of a bioavailability enhancer comprising an inhibitor of cytochrome P450, or an inhibitor of P-glycoprotein mediated membrane transport. The prior art by Sahana D K et al (Journal of Pharmaceutical Sciences, 2008, 97(4), 1530-42) discloses PLGA nanoparticles for oral delivery of hydrophobic drugs, in particular the influence of the organic solvent on nanoparticle formation and release behavior *in vitro* and *in vivo*, using estradiol as a model drug.

The present invention provides a simple and mild process for the preparation of nano- and/or microparticles containing a therapeutic agent, which process allows to enhance the solubility of poorly water soluble therapeutic agents, to target specific sections of the gastrointestinal tract (GIT), and/or to reduce the first- pass effect, thus giving rise to an increased bioavailability. Moreover, the invention provides pharmaceutical formulations comprising the nano- and/or microparticles obtainable from the process.

Thus, in a first aspect, the invention relates to a process for the production of nano- and/or microparticles for use in oral administration, which particles contain one or more therapeutic agents dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof,
said process comprising the steps of
a) providing a solution containing
   a1) the one or more therapeutic agents in dissolved form;
   a2) the one or more polymers in dissolved form, and
   a3) a solvent mixture containing (i) at least one solvent S1 which is fully miscible with water, and which is a solvent for the one or more therapeutic agents with a solubility of the therapeutic agent(s) in the solvent S1 being 10g/l or higher at 20°C, and a solvent for the one or more polymers, with a solubility of the polymer(s) in the solvent S1 being 100g/l or higher at 20°C, and (ii) at least one solvent S2 which is fully miscible with solvent S1 and which is partially miscible with water, with a solubility of the solvent S2 in water being 1 to 60 wt% of the solvent S2 in relation to the total weight of a mixed phase containing water and the solvent S2 at 20°C;
b) adding an aqueous surfactant solution with a volume of at least 2 times the volume of the solution provided in step a) to the solution provided in step a) while the solution provided in step a) is stirred; and
c) allowing the nano- and/or microparticles to form via extraction of the solvents S1 and S2 into the aqueous surfactant solution.

In a second aspect, the invention relates to a pharmaceutical formulation for oral administration comprising nano- and/or microparticles, which particles contain one or more therapeutic agents dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, which pharmaceutical formulation is obtainable by the above-mentioned process.

The invention is defined by the claims. Any subject-matter falling outside the scope of the claims is provided for information purposes only.

The process according to the present invention provides convenient control of the particle size and the particle size distribution. It can be used to produce nano- and/or microparticles, e.g. in the size range from 10 nm to 1000 µm. The process can be carried out as a simple one-pot process and a may readily be scaled up to meet commercial-scale production needs. Moreover, it is very efficient in that it enables a reduction of the energy and the time required for the production of the particles. In addition, comparably small amounts of solvents and surfactants as well as toxicologically acceptable solvents can be conveniently used.

Typically, the particle size of the nano- and/or microparticles in the context of the present invention, as determined e.g. by laser scattering, ranges from 10 nm to 1000 µm, preferably from 50 nm to 500 µm, and especially 50 nm to 100 µm. The d₉₀ value, determined via laser scattering on a particle number basis, is typically below 100 µm, preferably below 10 µm. As will be understood, the reference to "nano- and/or microparticles" indicates that the particles may be completely or predominantly in the nanometer size range (such as 10 to 100 nm), that they may be completely or predominantly in the micrometer size range (such as 0.1 to 1000 µm, or preferably 0.1 to 100 µm), or that particle mixtures of nano- and microparticles can be prepared and can be used in the context of the invention. It is an advantage of the nano- and/or microparticles obtainable by the process in accordance with the invention that they are generally non-agglomerating. Preferably, the nano- and/or microparticles are nano- and/or microspheres.

The process in accordance with the invention is not particularly limited with respect to the therapeutic agent(s) contained in the nano- and/or microparticles, and is very versatile with respect to the nature of agent to be dispersed in the polymer matrix. However, the advantages of the process of the invention are most pronounced for therapeutic agents classified in class II or III in at least one of the BCS classification system (e.g. Wu and Benet, Pharm Res 22: 11 -23 (2005)) or the BDDCS classification system.

Further, this technology is advantageous for drugs with short biological half life. Here, targeted delivery to the colon circumvents the first pass metabolism as it is observed for the normal oral application. Thus, the classification for therapeutic agents is not limited to those of class II or III as mentioned above.

Exemplary therapeutic agents for use in the context of the present invention are raloxifen, atorvastatine, rosuvastatin, quetiapine, duloxetine, asenapine, aripiprazole, methotrexate, glucagon, vasopressin, allendronic acid, clodronic acid, ezetimibe, valsartan, olmesartan, telmisartan, irbesartan, candesartan, bosentan, cytarabine, doxorubicin, irinotecan, diltiazem, verapamil, cortisol, estradiol, progesterone, tamoxifen, morphine, bubrenorphine, selegiline, risedronic acid, terbutaline, tiludronic acid, zoledronic acid, ziprasidone, naloxone, pamidronic acid, etidronic acid, albendazole, amidrine, carvedilol, paclitaxel, docetaxel, mesalazine, budesonide, prednisone, paracetamol, dexamethasone, omeprazole, risperidone, L-Dopa, diclofenac, metoprolol, bleomycin, perindopril, trandolapril, ramipril, cilazapril, moexipril, spirapril, fluorouracil, ibuprofen, nifedipine, ondansetron, rivastigmine, simvastatin, losartan, eprosartan, lisinopril and captopril, including, where applicable, pharmaceutically acceptable salt forms or prodrug forms thereof. Preferred therapeutic agents for use in the context of the invention are selected from raloxifen, atorvastatine, rosuvastatin, quetiapine, duloxetine and asenapine, including pharmaceutically acceptable salt forms thereof.

The nano- and/or microparticles may contain a single therapeutic agent, or a combination of two or more therapeutic agents. Thus, unless defined otherwise in, or dictated by, a specific context, the generic reference to a therapeutic agent herein encompasses the possibility that two or more therapeutic agents are used.

As will be understood by the skilled person, pharmaceutically acceptable salt forms may be formed, e.g., by protonation of an atom carrying an electron lone pair which is susceptible to protonation, such as an amino group, with an inorganic or organic acid, or as a salt of a carboxylic acid group with a physiologically acceptable cation as they are well known in the art. Exemplary base addition salts comprise, for example, alkali metal salts such as sodium or potassium salts; alkaline-earth metal salts such as calcium or magnesium salts; ammonium salts; aliphatic amine salts such as trimethylamine, triethylamine, dicyclohexylamine, ethanolamine, diethanolamine, triethanolamine, procaine salts, meglumine salts, diethanol amine salts or ethylenediamine salts; aralkyl amine salts such as N,N-dibenzylethylenediamine salts, benetamine salts; heterocyclic aromatic amine salts such as pyridine salts, picoline salts, quinoline salts or isoquinoline salts; quaternary ammonium salts such as tetramethylammonium salts, tetraethylammonium salts, benzyltrimethylammonium salts, benzyltriethylammonium salts, benzyltributylammonium salts, methyltrioctyl-ammonium salts or tetrabutylammonium salts; and basic amino acid salts such as arginine salts or lysine salts. Exemplary acid addition salts comprise, for example, mineral acid salts such as hydrochloride, hydrobromide, hydroiodide, sulfate salts, nitrate salts, phosphate salts (such as, e.g., phosphate, hydrogenphosphate, or dihydrogenphosphate salts), carbonate salts, hydrogencarbonate salts or perchlorate salts; organic acid salts such as acetate, propionate, butyrate, pentanoate, hexanoate, heptanoate, octanoate, cyclopentanepropionate, undecanoate, lactate, maleate, oxalate, fumarate, tartrate, malate, citrate, nicotinate, benzoate, salicylate, pamoate or ascorbate salts; sulfonate salts such as methanesulfonate, ethanesulfonate, 2-hydroxyethanesulfonate, benzenesulfonate, p-toluenesulfonate (tosylate), 2-naphthalenesulfonate, 3-phenylsulfonate, or camphorsulfonate salts; and acidic amino acid salts such as aspartate or glutamate salts.

Prodrugs that can be used in the present invention are generally pharmaceutically acceptable derivatives which have chemically or metabolically cleavable groups and yield, by solvolysis or under physiological conditions, the agents which are pharmaceutically active in vivo. Prodrugs of agents that can be used in the present invention may be formed in a conventional manner with a functional group of the compounds such as with an amino, hydroxy or carboxy group. Prodrugs include acid derivatives well known to the person skilled in the art, such as, for example, esters prepared by reaction of the parent acidic compound with a suitable alcohol, or amides prepared by reaction of the parent acid compound with a suitable amine. When an agent employed in the present invention, in particular a compound of the general formula (I), has a carboxyl group, an ester derivative prepared by reacting the carboxyl group with a suitable alcohol or an amide derivative prepared by reacting the carboxyl group with a suitable amine is exemplified as a prodrug. An especially preferred ester derivative as a prodrug is methylester, ethylester, n-propylester, isopropylester, n-butylester, isobutylester, tert-butylester, morpholinoethylester or N,N-diethylglycolamido-ester. When a compound employed in the present invention has a hydroxy group, an acyloxy derivative prepared by reacting the hydroxyl group with a suitable acylhalide or a suitable acid anhydride is exemplified as a prodrug. An especially preferred acyloxy derivative as a prodrug is -OC(=O)-CH₃, -OC(=O)-C₂H₅, -OC(=O)-C₃H₇, -OC(=O)-(tert-butyl), -OC(=O)-C₁₅H₃₁, -OC(=O)-CH₂CH₂COONa, -O(C=O)-CH(NH₂)CH₃ or -OC(=O)-CH₂-N(CH₃)₂. When an agent employed in the present invention has an amino group, an amide derivative prepared by reacting the amino group with a suitable acid halide or a suitable mixed anhydride is exemplified as a prodrug. An especially preferred amide derivative as a prodrug is -NHC(=O)-(CH₂)₂OCH₃ or -NHC(=O)-CH(NH₂)CH₃.

The therapeutic agent is usually contained in the nano- and/or microparticles in an amount of 1 to 40% by weight, preferably 5 to 20% by weight, based on the total weight of the nano- and/or microparticles.

The therapeutic agent is dispersed in a non-crystalline form in the matrix provided by the nano- and/or microparticles. In particular, the therapeutic agent may be dispersed in the matrix as an amorphous solid, or may form a solid solution, i.e. may be present in the form of a molecular dispersion, in the matrix. Typically, no crystalline form of the therapeutic agent is present in the nano- and/or microparticles. The presence of the non-crystalline form or absence of a crystalline form of the therapeutic agent may be confirmed, e.g., via X-ray diffraction. Typically, the nano- and/or microparticles obtainable by the process in accordance with the invention contain the therapeutic agent dispersed across their full cross-section. However, if the process includes a step of coating the nano- and/or microparticles initially prepared, the coating may be free of a therapeutic agent.

The nano- and/or microparticles provide a matrix wherein the therapeutic agent is dispersed. Preferably, the nano- and/or microparticles consist of this matrix and the therapeutic agent, or of the matrix, the therapeutic agent, and an optional coating applied to the surface of the material(s) forming the matrix. The matrix contains, as a matrix component, one or more polymers selected from
(i) cellulose ethers and cellulose esters,
(ii) copolymers of methacrylic acid with one or more (meth)acrylic acid esters,
(iii) copolymers of two or more (meth)acrylic acid esters,
and mixtures thereof. The matrix may contain, as further matrix components, other polymers and/or non-polymeric excipients. Typically, the content of the one or more polymers (i) to (iii) as defined above in the matrix is 50% by weight or more, preferably 70% by weight or more, more preferably 80% by weight or more, and in particular 90% by weight or more, based on the total weight of the matrix (i.e. excluding the weight of the therapeutic agent, and of an optional coating). In addition, it is preferred that the one or more polymers (i) to (iii) as defined above represent the only polymers in the matrix. It is more preferred that the matrix consists of one or more polymers selected from polymers (i) to (iii), and it is especially preferred that the nano- and/or microparticles consist of one or more polymers selected from polymers (i) to (iii) and the therapeutic agent, or of one or more polymers selected from polymers (i) to (iii), the therapeutic agent, and a coating applied to the surface of the polymers selected from polymers (i) to (iii).

In the context of the present invention, it is generally preferred to use polymers in the matrix provided by the nano- and/or microparticles that show a pH dependent solubility in water or water to which a buffer has been added for adjusting the pH value. In particular, it is advantageous for the provision of particles targeting of the colon if the polymers are soluble at pH values above pH 5.0, preferably at a pH value in the range of 5.0 to 7.5, and in particular of 5.0 to 7.0. As an illustration, the following table lists threshold pH values as the pH values above which the concerned polymers are soluble in water or water to which a buffer has been added for adjusting the pH value, for exemplary polymers. They are reported e.g in S. Thakral et al., Expert Opin. Drug Deliv. 2013 10(1); Evonik Industries AG, EUDRAGIT Application Guidelines, 12th edition; or Harke Pharma, Product brochure Products and Services.

**Table 1: Threshold pH-values for exemplary polymers**

| **Polymer** | **Threshold pH** |
|---|---|
| Eudragit L 100 | 6.0 |
| Eudragit S100 | 7.0 |
| Eudragit L30D | 5.6 |
| Eudragit L 100-55 | 5.5 |
| FS30D | 6.8 |
| | |
| HPMCP HP-50 | 5.0 |
| HPMCP HP-55 | 5.5 |
| HPMCP HP-55S | 5.5 |
| HPMC AS-LG | 5.5 |
| HPMC AS- MG | 6.0 |
| HPMC AS- HG | 6.5 |

As noted above, one type of polymer that may be contained in the matrix provided by the nano- and/or microparticles are cellulose ethers or cellulose esters. Cellulose ethers are cellulose derivatives wherein a part or all of the hydroxyl groups contained in the cellulose have been etherified. Typical examples are alkyl ethers, in particular C1-C8 alkyl ethers, and more preferably C1-C6 alkyl ethers, such as methyl ethers, ethyl ethers or propyl ethers; or hydroxyalkyl ethers, in particular C2-C8 monohydroxyalkyl ethers, and more preferably C2-C6 monohydroxyalkyl ethers such as hydroxyethyl or hydroxypropyl ethers. It should be understood that the term "cellulose ether" as used in the context of the present invention encompasses celluloses with different ether groups, such as ethyl methyl cellulose, or hydroxypropyl methyl cellulose, and celluloses containing ether groups formed with a hydroxyl group of the cellulose together with other functional groups, such as an ester group. It also encompasses the possibility that a hydroxyl group of the cellulose is replaced by an ether group -O-R, wherein the group R further contains an ester bond, as this is the case e.g. in hydroxypropyl methyl cellulose acetate succinate (HPMC AS). Celluloses wherein different OH groups of the cellulose molecule have been derivatized by ether and ester groups may be referred to herein as cellulose ethers as well as cellulose esters.

Cellulose esters are cellulose derivatives wherein a part or all of the hydroxyl groups of the cellulose molecules have been esterified. Typically, esters are formed with monocarboxylic acids or dicarboxylic acids, preferably C2-C8 monocarboxylic or dicarboxylic acids. Examples are esters with acetic acid, phthalic acid or succinic acid. It should be understood that the term "cellulose ester" as used in the context of the present invention encompasses celluloses with different ester groups, and celluloses containing ester groups formed with a hydroxyl group of the cellulose together with other functional groups, such as an ether grous. Celluloses wherein different OH groups of the cellulose molecula have been derivatized by ether and ester groups may be referred to herein as cellulose ethers as well as cellulose esters.

Preferred cellulose ethers or esters in the context of the invention are selected from methyl cellulose, ethyl cellulose, propyl cellulose, ethyl methyl cellulose, hydroxyethyl cellulose, hydroxypropyl cellulose, hydroxypropyl methyl cellulose, hydroxyethyl methyl cellulose, hydroxyethyl ethyl cellulose, cellulose acetate phthalate (CAP), cellulose acetate, hydroxypropyl methyl cellulose phthalate (HPMCP), hydroxypropyl methylcellulose acetate succinate (HPMC AS) and mixtures thereof. Particularly preferred are ethylcellulose, CAP, HPMCP or HPMC AS.

Another type of polymer that may be contained in the matrix provided by the nano- and/or microparticles are copolymers of methacrylic acid with one or more (meth)acrylic acid esters. As will be understood, the term (meth)acrylic acid esters refers to acrylic acid esters as well as methacrylic acid esters. Preferred (meth)acrylic acid esters are C1-C6 alkyl esters, such as methyl, ethyl or propyl ethers, and in particular methyl or ethyl esters. The copolymers comprise monomer units formed by methacrylic acid and by one or more (meth)acrylic acid esters in an amount of 50% or more, preferably 70% or more, more preferably 80% or more, and in particular 90% or more, based on the total number of polymerized units. Most preferred are copolymers consisting of units formed by methacrylic acid and one or more (meth)acrylic acid esters. It is generally preferred that the methacrylic acid accounts for at least 5% of the number of polymerized units in the copolymers of methacrylic acid with one or more (meth)acrylic acid esters.

Especially preferred are binary copolymers of a methacrylic acid with one (meth)acrylic acid ester, or ternary copolymers of a methacrylic acid with two (meth)acrylic acid esters, and in both cases, the most preferred (meth)acrylic acid ester is the methyl or the ethyl ester. The amount (in terms of mol%, based on the total amount of polymerized units) of the comonomer units in the binary copolymers is preferably 5 to 90%, more preferably 10 to 70% and in particular 30 to 50% methacrylic acid and preferably 95 to 10%, more preferably 90 to 30%, and in particular 50 to 70% (meth)acrylic acid ester. The amount (in terms of mol%, based on the total amount of polymerized units) of the comonomer units in the ternary copolymers is preferably 5 to 50%, more preferably 5 to 30% methacrylic acid, and preferably 95 to 50%, more preferably 95 to 70%, of the other two (meth)acrylic acid esters.

Preferred as specific copolymers of methacrylic acid with one or more (meth)acrylic acid esters for use in the context of the invention are poly(methacrylic acid-co-methyl methacrylate), poly(methacrylic acid-co-methyl acrylate), poly(methacrylic acid-co-ethyl acrylate), poly(methacrylic acid-co-ethyl methacrylate), and poly(methyl acrylate-co-methyl methacrylate-co-methacrylic acid), more preferred are poly(methacrylic acid-co-methyl methacrylate), e.g. in a (molar) monomer unit ratio of 2:1 to 1:2, especially 1:1 to 1:2, poly(methacrylic acid-co-ethyl acrylate), e.g. in a (molar) monomer unit ratio of 2:1 to 1:2, especially 1:1, or poly(methyl acrylate-co-methyl methacrylate-co-methacrylic acid), e.g. in a (molar) monomer unit ratio of 7:3:1.

Such copolymers of methacrylic acid with one or more (meth)acrylic acid esters are commercially available e.g. as Eudragit® polymers, such as Eudragit® L100, L100-55, S100 or FS30D.

Still another type of polymer that may be contained in the matrix provided by the nano- and/or microparticles are copolymers of two or more (meth)acrylic acid esters, and mixtures thereof. Also in this context, the term (meth)acrylic acid esters refers to acrylic acid esters as well as methacrylic acid esters. Preferred (meth)acrylic acid esters are C1-C6 alkyl esters, such as methyl, ethyl or propyl ethers, and in particular methyl or ethyl esters. Also preferred are copolymers formed from at least one (meth)acrylic acid C1-C6 alkyl ester in combination with a (meth)acrylic acid C1-C6 alkyl ester wherein a functional group with a positive charge, e.g. a trialkylammonium group, is additionally covalently bound to the C1-C6 alkyl group of the ester. An example of such a positively charged (meth)acrylic acid comonomer is trimethylammonioethyl methacrylate, e.g. in the form of its chloride. The copolymers comprise monomer units formed by two or more (meth)acrylic acid esters in an amount of 50% or more, preferably 70% or more, more preferably 80% or more, and in particular 90% or more, based on the total number of polymerized units. Most preferred are copolymers consisting of units formed by two or more (meth)acrylic acid esters.

Especially preferred are binary copolymers of an acrylic acid C1-C6 alkyl ester and a methacrylic acid C1-C6 alkyl ester, binary copolymers of a (meth)acrylic acid C1-C6 alkyl ester and a (meth)acrylic acid C1-C6 alkyl ester carrying a trialkylammonium group attached to the C1-C6 alkyl group, or ternary copolymers of two (meth)acrylic acid C1-C6 alkyl esters and a (meth)acrylic acid C1-C6 alkyl ester carrying a trialkylammonium group attached to the C1-C6 alkyl group. In all cases, the most preferred (meth)acrylic acid ester is the methyl or the ethyl ester. Preferred trialkylammonium groups carry methyl and/or ethyl groups as alkyl groups. The amount (in terms of mol%, based on the total amount of polymerized units) of the comonomer carrying a trialkylammonium group, where applicable, is preferably 1 to 10%, the balance being the one or more (meth)acrylic acid C1-C6 alkyl ester units.

Preferred as specific copolymers of one or more (meth)acrylic acid esters for use in the context of the invention is poly(ethyl acrylate-co-methyl methacrylate), e.g. in a (molar) monomer unit ratio of 1 to 2 moles of the ethyl acrylate units to 1 to 2 moles of the methyl methacrylate, and in particular 2:1, poly(ethyl acrylate-co-methyl methacrylate-co-trimethylammonioethyl methacrylate chloride), e.g. in a (molar) monomer unit ratio of 1 to 2 moles of the ethyl acrylate units to 1 to 2 moles of the methyl methacrylate units to 0.1 to 0.2 moles of the trimethylammonioethyl methacrylate chloride units, and in particular 1:2:0.1 or 1:2:0.2.

Such copolymers of methacrylic acid with one or more (meth)acrylic acid esters are also commercially available e.g. as Eudragit® polymers, such as Eudragit® NE 30 D, NE 40 D, NM 30 D, RL100 or RS100.

In accordance with the process of the invention, a solution is formed which contains the therapeutic agent(s) in dissolved form, and the one or more polymers, i.e. the one or more polymers selected from (i) to (iii) discussed above, in dissolved form. Typically, the solution is a single phase solution. It will be understood that, in cases where the matrix provided by the nano- and/or microparticles contains other matrix components than the polymers selected from polymers (i) to (iii) and the therapeutic agent, the/these other component(s) should preferably be present also in dissolved form in this solution.

The solvent mixture used to prepare this solution contains at least one solvent S1 and at least one solvent S2, which will be discussed in further detail below. Unless indicated otherwise, or dictated by a specific context, any reference to solvent S1 or solvent S2 is intended to include the option that more than one of S1 or S2, respectively, is used. Further solvents or water may be present in the solution in addition to S1 and S2, as far as they do not have a negative impact on the process. However, it is generally preferred that the solvent mixture used to prepare the solution in step a) of the process in accordance with the invention contains at least 80, more preferably at least 90% (vol./vol., based on the total volume of solvents used) of S1 and S2, and it is most preferred that the solvent mixture consists of S1 and S2. Moreover, it is generally preferred that no halogenated solvents are used in the process of the invention as solvent S1, S2 or as an additional solvent.

As solvent S1, a solvent is selected which is a solvent for the one or more therapeutic agents to be contained in the nano- or microparticles. In the present invention, the solubility of the therapeutic agent(s) in the solvent S1 is 10 g/l or higher at 20 °C. In this context, the solubility is indicated as the weight of the dissolved substance (the solute) per volume of the solvent (i.e. the volume of solvent added to the solute).

The solubilities of a therapeutic agent in diverse solvents are reported in the literature or can be tested in a straightforward manner. Nevertheless, the following tables provide additional orientation regarding the solubility of preferred therapeutic agents. As will be understood, the term "soluble" indicates that the concerned solvent acts as a solvent for the concerned therapeutic agent.

**Tables 2 to 4: solubilities of exemplary therapeutic agents**

| Solubility of Asenapine maleate | |
|---|---|
| Solvent | |
| DMSO | soluble |
| NMP | soluble |
| Benzylalcohol | soluble |
| Ethyl acetate | soluble |
| Ethyl formate | soluble |
| Methyl acetate | soluble |
| Ethanol | soluble |
| Iso propanol | non-soluble |
| Glycofurol | soluble |
| EMK | soluble |
| Acetone | soluble |
| THF | soluble |
| Dimethylcarbonate | soluble |
| Isopropyl acetate | non-soluble |
| Isopropyl formate | soluble |

| Solubility of Duloxetine HCl | |
|---|---|
| Solvent | |
| DMSO | soluble |
| NMP | soluble |
| Benzylalcohol | soluble |
| Ethyl acetate | non-soluble |
| Ethyl formate | non-soluble |
| Methyl acetate | non-soluble |
| Methyl formate | soluble |
| Ethanol | soluble |
| Glycofurol | soluble |
| Triacetin | non-soluble |
| EMK | non-soluble |
| Acetone | non-soluble |
| THF | soluble |
| Methyl benzoate | non-soluble |
| Benzyl benzoate | non-soluble |

| Solubility of Rosuvastatin Ca | |
|---|---|
| Solvent | |
| DMSO | soluble |
| NMP | soluble |
| Benzylalcohol | soluble |
| Ethyl acetate | soluble |
| Ethyl formate | soluble |
| Methyl acetate | soluble |
| Methyl formate | soluble |
| Ethanol | non-soluble |
| Glycofurol | soluble |
| Triacetin | non-soluble |
| EMK | soluble |
| Acetone | soluble |
| THF | soluble |
| Isopropyl acetate | non-soluble |
| Isopropyl formate | non-soluble |

**Tables 5 to 7: solubilities of exemplary therapeutic agents**

| Solubility of Quetiapine fumarate | |
|---|---|
| Solvent | |
| DMSO | soluble |
| NMP | soluble |
| Benzylalcohol | soluble |
| Ethyl acetate | non-soluble |
| Ethyl formate | non-soluble |
| Methyl acetate | non-soluble |
| Methyl formate | non-soluble |
| Ethanol | non-soluble |
| Glycolurol | soluble |
| Triacetin | non-soluble |
| EMK | non-soluble |
| Acetone | non-soluble |
| THF | soluble |

| Solubility of Raloxifene HCl | |
|---|---|
| Solvent | |
| DMSO | soluble |
| NMP | soluble |
| Benzylalcohol | non-soluble |
| Ethyl acetate | non-soluble |
| Ethyl formate | non-soluble |
| Methyl acetate | non-soluble |
| Methyl formate | non-soluble |
| Ethanol | non-soluble |
| EMK | non-soluble |
| Acetone | non-soluble |
| THF | non-soluble |
| Dichloromethane | non-soluble |
| Isopropyl acetate | non-soluble |
| Isopropyl formate | non-soluble |
| Propyl formate | non-soluble |
| Benzyl formate | non-soluble |

| Solubility of Aripiprazole | |
|---|---|
| Solvent | |
| DMSO | soluble |
| NMP | soluble |
| Benzyl alcohol | soluble |
| Benzyl benzoate | soluble |
| Ethyl acetate | soluble |
| Ethyl formate | soluble |
| Methyl acetate | soluble |
| Methyl formate | non-soluble |
| Isopropyl acetate | non-soluble |
| Isopropyl formate | soluble |
| Ethanol | non-soluble |
| EMK | soluble |
| Acetone | soluble |
| Glycofurol | soluble |
| Triacetin | non-soluble |
| THF | soluble |

In addition, the solvent S1 is a solvent for the one or more polymers selected from polymers (i) to (iii) above. It will be understood that it is preferably also a solvent for any other optional matrix components contained in the matrix provided by the nano- and/or microparticles. In the present invention, the solubility of the one or more polymers selected from (i) to (iii) in the solvent S1 is 100 g/l or higher at 20 °C as the weight of the dissolved substance (the solute) per volume of the solvent (i.e. the volume of solvent added to the solute).

As for the required full miscibility of solvent S1 with water, the term "fully miscible" is used herein in line with conventional practice to indicate that S1 and water can be mixed (typically at room temperature, e.g. 20°C) in all proportions to prepare a stable single phase mixture.

The solubilities of polymers, especially polymers approved for use in pharmaceutical formulations, in diverse solvents are reported in the literature or can be tested in a straightforward manner. Still, the following tables provide further orientation regarding the solubility of preferred polymers. As will be understood, the term "soluble" indicates that the concerned solvent acts as a solvent for the concerned polymer. The same applies for the miscibility or solubility of solvents in water, for which values can be derived from numerous standard collections of physical and chemical data, such as the CRC Handbook of Chemistry and Physics, Taylor & Francis. Also in this respect, the following tables provide an additional overview. As will be understood, the value "100%" indicated in table 10 means that the concerned solvent is miscible with water in all proportions.

**Tab. 8: Solubility of cellulose ether or ester polymers**

| | Ethylcellulose | HPMPC | HPMC AS | Cellulose Acetate Phthalate |
|---|---|---|---|---|
| Ethyl formate | slightly soluble | - | - | non-soluble |
| Ethyl acetate | slightly soluble | - | non-soluble | non-soluble |
| Methyl acetate | slightly soluble | - | - | soluble |
| DMSO | non-soluble | soluble | soluble | soluble |
| NMP | soluble | soluble | soluble | soluble |
| Acetone | non-soluble | - | - | - |
| Glycofurol | non-soluble | - | - | soluble |
| Ethanol | slightly soluble | non-soluble | non-soluble | non-soluble |
| EMK | slightly soluble | - | - | soluble |
| THF | soluble | - | - | - |
| Dichloromethane | n.a. | slightly soluble | - | non-soluble |

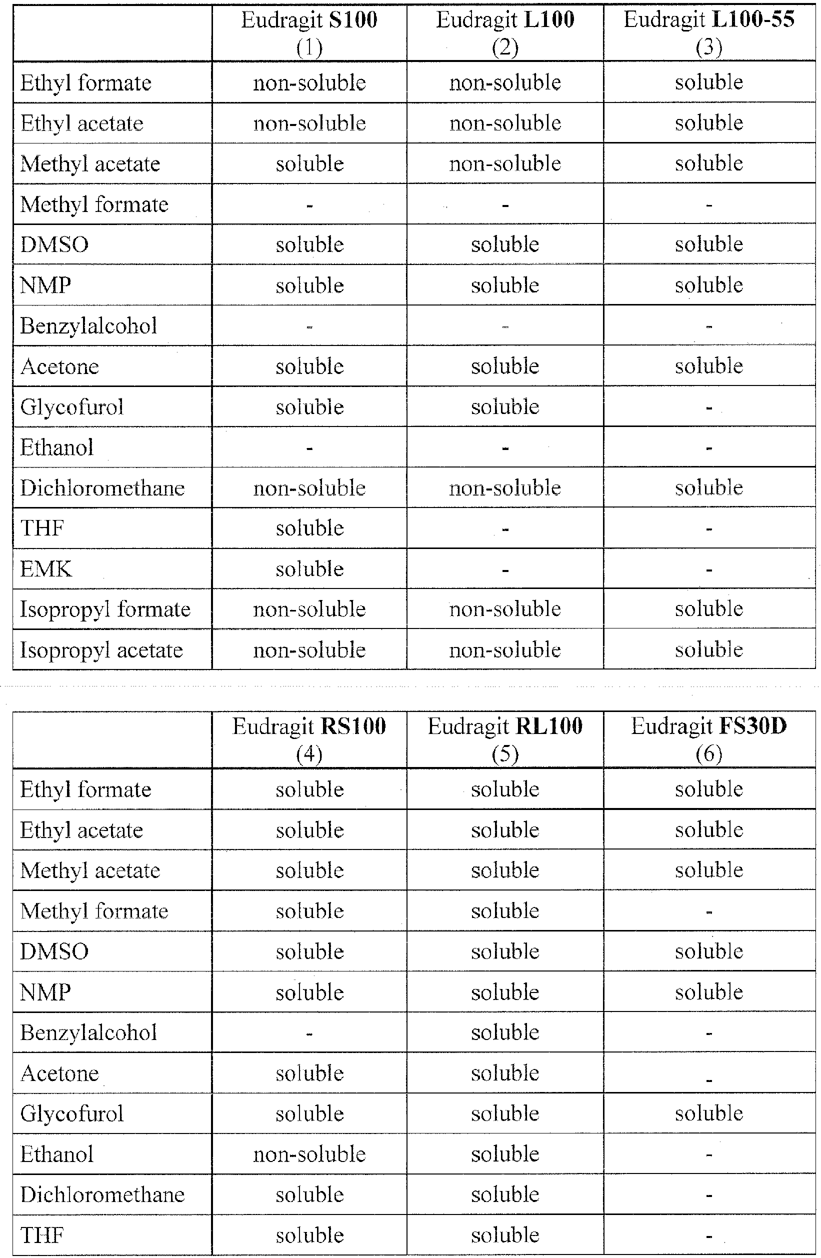

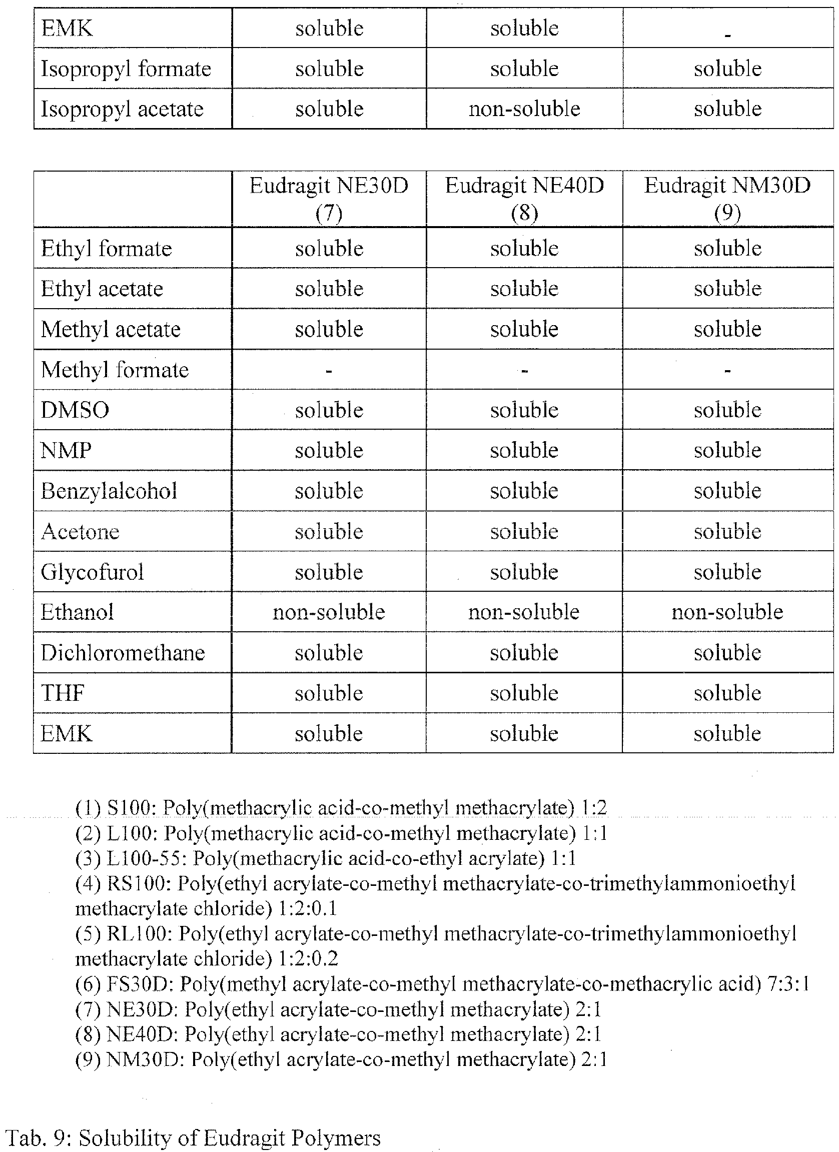

**Tab. 10: Boiling point and solubility in water of organic solvents**

| | Boiling point* [°C] | Solubility in water **[g/L] (20°C) |
|---|---|---|
| Ethyl formate | 54.5 | 105*** |
| Ethyl acetate | 77.06 | 85.3 |
| Methyl acetate | 57 | 319 |
| Methyl formate | -31.5 | 300 |
| Aceton | 56.2 | soluble |
| Butyl acetate | 126.5 | 7 |
| tert. Butyl acetate | 97 - 98 | insoluble |
| Butyl formate | 106.8 | slow decomposition |
| Isobutyl formate | 98.4 | n.a.- |
| tert. Butyl formate | 97 | n.a. |
| n-Propyl acetate | 101.6 | 21.2 |
| Isopropyl acetate | 90 | 31 |
| n- Propyl formate | 81.3 | 28 |
| Isopropyl formate | 68.2 | slow decomposition |
| Dichlormethan | 40 | 20 |
| DMSO | 189 | 1000 |
| NMP | 202 | 1000 |
| THF | 67 | soluble |
| Benzylalcohol | 205.3 | 40 |
| Glycofurol | 328 | soluble |
| Methylisopropylketone | 94-95 | 6 |
| Ethylmethylketone (EMK) | 79.6 | 292 |
| Dimethyl Carbonate | 90-91 | 139 |

| | | |
|---|---|---|
| * Handbook of Chemistry and Physics, CRC Press, 65th edition, 1984- 1985 ** Merck Chemicals Product Information *** Gestis Stoffdatenbank | | |

Preferred solvents S1 are selected from acetone, dimethyl sulfoxide (DMSO), N-methylpyrrolidone (NMP), glycofurol, tetrahydrofurane, ethanol and mixtures of two or more thereof. Especialy preferred solvents are DMSO, NMP, glycofurol or mixtures of two or three thereof.

Solvent S2 for use in the process of the invention is fully miscible with solvent S1, i.e. both solvents can be mixed in any proportion without phase separation. Moreover, it is partially miscible with water. In the present invention, the solubility of S2 in water is 1 to 60 wt% at 20°C (as wt% of the solvent S2 in relation to the total weight of the mixed phase containing water and the solvent S2), more preferably 2 to 40 wt%. As noted above, such solubility values are derivable from literature, and additional guidance is provided by the above tables.

Preferred solvents S2 are selected from alkyl acetates, especially C1-C3 alkyl acetates, alkyl formates, especially C1-C3 alkyl formates, dimethyl carbonate, ethyl methyl ketone and mixtures of two or more thereof. Particularly preferred are solvents S2 selected from ethyl acetate, methyl acetate, ethyl formate, propyl formate, and mixtures of two or more thereof.

The volume ratio of solvent S1 to solvent S2 in the solution provided in step a) of the process of the invention, is preferably 5-50 vol% S1 to 50-95 vol% S2, based on the sum of the volumes S1+S2 as 100 vol%. Particularly preferred are ratios of 20-50 vol% S1 to 50-80 vol% S2.

For further illustration, the following table lists the solubility of preferred polymers in certain solvent mixtures.

**Table 11**

| Solvents (ratio vol:vol) | Eudragit **S100** (1) | Eudragit **L100** (2) | Eudragit **L100-55** (3) | Eudragit **RS100** (4) | Eudragit **RL100** (5) |
|---|---|---|---|---|---|
| Ethyl acetate/DMSO 70:30 | - | non-soluble | - | - | - |
| Ethyl acetate/DMSO 60:40 | soluble | soluble | soluble | soluble | soluble |
| Ethyl acetate/NMP 70:30 | - | non-soluble | soluble | - | - |
| Ethyl acetate/NMP 60:40 | soluble | soluble | soluble | soluble | soluble |
| Ethyl acetate/Glycofurol 70:30 | - | non-soluble | - | - | - |
| Ethyl acetate/Glycofurol 60:40 | soluble | soluble | - | - | - |
| Ethyl formate/DMSO 70:30 | - | soluble | - | - | - |
| Ethyl formate/DMSO 60:40 | soluble | soluble | - | - | - |
| Ethyl formate/NMP 70:30 | - | soluble | - | - | - |
| Ethyl formate/NMP 60:40 | - | soluble | - | - | - |
| Ethyl formate/Glycofurol 70:30 | - | soluble | - | - | - |
| Ethyl formate/Glycofurol 60:40 | soluble | soluble | - | - | - |
| Isopropyl formate/DMSO 60:40 | soluble | - | - | - | - |
| Isopropyl formate/NMP 60:40 | soluble | - | - | - | - |
| Isopropyl formate/Glycofurol 70:30 | soluble | - | - | - | - |
| Isopropyl formate/Glycofurol 60:40 | soluble | - | - | - | - |
| Isopropyl acetate/DMSO 60:40 | non-soluble | - | - | - | - |
| Isopropyl acetate/NMP 60:40 | non-soluble | - | - | - | - |
| Isopropyl acetate/Glycofurol 70:30 | soluble | - | - | - | - |
| Isopropyl acetate/Glycofurol 70:30 | soluble | - | - | - | - |
| Methyl acetate/DMSO 95:5 | soluble | - | - | soluble | - |
| Methyl acetate/NMP 95:5 | soluble | - | - | soluble | - |
| Methyl acetate/Aceton 95:5 | non-soluble | - | - | soluble | - |
| Methyl acetate/ Glycofurol 95:5 | non-soluble | - | - | soluble | - |
| Methyl acetate/PEG300 95:5 | non-soluble | - | - | soluble | - |
| Methyl acetate/DMSO 93:7 | - | - | non-soluble | - | - |
| Methyl acetate/NMP 93:7 | - | - | non-soluble | - | - |
| Methyl acetate/Aceton 93:7 | - | - | non-soluble | - | - |
| Methyl acetate/ Glycofurol 93:7 | - | - | non-soluble | - | - |
| Methyl acetate/PEG300 93:7 | - | - | non-soluble | - | - |
| Methyl acetate/DMSO/Ethanol 92:6:2 | | non-soluble | | | |
| Methyl acetate/NMP/Ethanol 92:6:2 | | non-soluble | | | |
| Methyl acetate/Aceton/Ethanol 92:6:2 | | non-soluble | | | |
| Methyl acetate/Glycofurol/Ethanol 92:6:2 | | non-soluble | | | |
| Methyl acetate/PEG300/Ethanol 92:6:2 | | non-soluble | | | |

| | | | | | |
|---|---|---|---|---|---|
| (1) S100: Poly(methacylic acid-co-methyl methacrylate) 1:2 (2) L100: Poly(methacylic acid-co-methyl methacrylate) 1:1 (3) L100-55: Poly(methacylic acid-co-ethyl acrylate) 1:1 (4) RS100: Poly(ethyl acrylate-co-methyl methacrylate-co-trimethylammonioethyl methacrylate chloride) 1:2:0.1 (5) RL100: Poly(ethyl acrylate-co-methyl methacrylate-co-trimethylammonioethyl methacrylate chloride) 1:2:0.2 | | | | | |

The procedure that can be used to provide the solution in step a) of the process in accordance with the invention is not particularly limited. Exemplary approaches include: first mixing solvents S1 and S2, and subsequently dissolving the therapeutic agent and the polymer in the mixture of solvents; first dissolving the therapeutic agent and the polymer in solvent S1, and subsequently adding solvent S2 to the solution; or dissolving the therapeutic agent in a part of the total volume of S1, dissolving the polymer in a mixture of the remaining part of S1 and S2, and mixing the solutions thus obtained. It is generally preferred to dissolve the therapeutic agent in all or a part of solvent S1 separately, before the dissolved therapeutic agent comes into contact with S2. Generally, an efficient procedure is to dissolve the polymer in a mixture of solvents S1 and S2, to separately dissolve the therapeutic agent in solvent S1, and to combine the solutions thus prepared.

In the solution prepared in step a) of the process in accordance with the invention, the concentration of the polymer selected from polymers (i) to (iii) as defined above is preferably 1 wt% to 40 wt%, based on the total weight of the solution. More preferably, the solution contains 5 wt% to 30 wt% of the polymer. The concentration of the therapeutic agent can be suitably choscn with a view to the desired drug load of the resulting particles discussed above. The process of the present invention is capable of incorporating the therapeutig agent into the nano- and/or microparticles with a high efficiency, such that a high ratio of typically 80 % (wt/wt) or more of the therapeutic agent dissolved in the solution a) will be incorporated into the particles.

After the solution containing the therapeutic agent and the polymer selected from polymers (i) to (iii) as discussed above has been provided in step a), an aqueous surfactant solution is added thereto. The addition of the aqueous surfactant solution to the solution provided in step a) is carried out while the solution provided in step a) is stirred. For example, the surfactant solution can be poured into the stirred solution provided in step a) over a period of 10 s to 5 min.

It is preferred for reasons of efficiency to prepare the solution of step a) in a vessel with a volume that is sufficiently large to additionally accommodate the volume of the surfactant solution to be added. In this case, the process can be carried out as a one-pot process, i.e. a process where the solution containing a therapeutic agent and a polymer as defined above, and the desired nano- and/or microparticels can be prepred in a single vessel in subsequent steps.

The aqueous surfactant solution contains water, optionally in combination with one or more organic solvents, such as ethyl alcohol or acetone. However, as implied by the term "aqueous", water is the main solvent in the aqueous surfactant solution with a volume ratio of more than 50 vol% of all solvents, preferably more than 70 vol%, and more preferably more then 90 vol%. It is particularly preferred that water is the only solvent in the aqueous surfactant solution.

The concentration of the surfactant in the aqueous surfactant solution is preferably in the range of 0.1% (w/v) to 30% (w/v), preferably 0.1% to 20%, and more preferably 0.1 to 5%, based on the total volume of the surfactant solution. As will be understood by the skilled reader, the concentration in weight by volume corresponds to the amount of the solute in g per 100 ml of the total volume of the solution, typically at 20°C.

Surfactants suitable for the aqueous surfactant solution encompass cationic-, anionic-, and non-ionic surfactants. Examples can be selected from polyoxyethylene-polyoxypropylene block copolymers, in particular polyoxyethylene-polyoxypropylene-polyoxyethylene-triblock copolymers such as Poloxamer®, Poloxamine®, polyethylene glycol alkyl ethers, fatty acid esters of polyoxyethylensorbitan, especially polyoxyethylenesorbitan monooleate and polyoxyethylenesorbitan monolaurate also referred to as polysorbates (Tween®, Span®), sucrose esters (Sisterna®, Ryoto Sugar Ester, Tokyo), gelatin, polyvinylpyrrolidone, fatty alcohol polyglycosides, Charps, Charpso, decyl-β-D-glycopyranoside, decyl-β-D-maltopyranoside, dodecyl-β-D-maltopyranoside, sodium oleate, polyethylene glycol, polyvinyl alcohol (PVA), polyethoxylated fatty acid ethers (Brij®), Triton X 100 or mixtures thereof. Preferred as a surfactant are polyvinyl alcohol, polyoxyethylene-polyoxypropylene-polyoxyethylene-triblock copolymers and fatty acid esters of polyoxyethylensorbitan, especially polyoxyethylenesorbitan monooleate and polyoxyethylenesorbitan monolaurate.

The aqueous surfactant solution may contain other components beside the water, optional additional solvents and the surfactant. In view of the fact that the polymers (i) to (iii) discussed above tend to have an increased solubility in aqueous solutions at high pH values, it is preferred that the pH value of the aqueous surfactant solution is 6 or less, more preferably 5 or less, and most preferably 4 or less. pH values of lower than 3 are typically not used. Such a pH can be conveniently adjusted by adding a buffer to the aqueous surfactant solution.

Moreover, it has been found to be advantageous if the aqueous surfactant solution added in step b) comprises a salt which dissolves in the water (such as NaCl), in order to increase the ionic strength. If such a salt is used, it is typically dissolved at a concentration of 1 to 5 g/l of the aqueous surfactant solution. Another optional component that may be contained in the aqueous surfactant solution added in step b) is a saccharide, preferably a mono- and/or disaccharide such as sucrose, which can modify the viscosity of the aqueous surfactant solution. If a saccharide is used, its concentration generally ranges from 1 to 50 g/l, preferably 5 to 30 g/l, in the aqueous surfactant solution. Also in this context, concentrations are given with a view to the amount of solute in relation to the volume of the solvent added.

In the process according to the invention, the aqueous surfactant solution is added in step b) to the solution provided in step a) in a volume which is at least two times the volume of the solution provided in step a). Preferably, the volume of the aqueous surfactant solution is at least 3 times that of the solution provided in step a). While it is possible to use very large volumes of the aqueous surfactant solution to prepare the nano- or microparticles, it is preferred to keep the volume at a low level in order to reduce the consumption of solvents and other components. Thus the volume of the aqueous surfactant solution is generally less than 5 times the volume of the solution provided in step a).

When the aqueous surfactant solution is added to the solution provided in step a) the aqueous surfactant solution forms the continuous phase in the resulting mixture, and acts at the same time as an extraction medium for the solvents S1 and S2 wherein the therapeutic agent and the polymer had been dissolved. Thus, a suspension of nano-or micro-particles forms spontaneously within minutes, or even less than a minute, upon addition of the aqueous surfactant solution to the solution provided in step a). The formation of the particles takes place without the need for any removal of a solvent from the mixture e.g. via evaporation. It has been found that the size and the size distribution of the nano- and/or microparticles can be conveniently controlled in this process, e.g. by varying the energy of agitation during the addition of the aqueous phase, or by varying the composition of the aqueous surfactant phase.

After the suspension of nano- and/or microparticles has been formed, they can be isolated from the liquid phase and dried via conventional methods, incuding e.g. extraction, spray drying, fluid bed drying, freeze drying, centrifugation, evaporation and/or filtration. These methods can also be used to remove remainders of the solvents S1 and/or S2, if necessary. Volatile solvents can be conveniently removed from the particles via evaporation. Less volatile solvents can be removed by other methods established in the art, such as extraction. Washing steps can also be added to the process of the invention as needed. A particularly convenient step in order to obtain a dry, reconstitutable powder containing the nano- and/or microparticles is the lyophilisation of the particles after their formation.

In accordance with a preferred embodiment of the process according to the invention, the nano- and/or microparticles formed in steps b) and c) are additionally coated with a polysaccharide. Such a coating can be applied to any of the nano- and/or microparticles in accordance with the invention. However, it is particularly preferred to apply it to those nano- and/or microparticles which do not contain a cellulose ether or ester in their matrix, i.e. those containing as polymer the polymer (ii) or (iii), but not (i) discussed above.

Such a coating can be applied by various procedures known to the person skilled in the art. A convenient way is to contact the nano- and/or microparticles with a solution of the polysaccharide, and subsequently removing the solvent to form the coating, e.g. by evaporation, lyophilisation or other conventional methods. Prior to the removal of the solvent, surplus solution not adhering to the surface of the nano- and/or microparticles can be removed, e.g. via filtration, centrifugation or other conventional methods. As a solvent for the coating solution containing the polysaccharide, water is preferred, but other solvents can be used as well. It will be understood that a solvent should preferably be used which does not substantially attack or dissolve the nano- and/or microparticles. For example, if water is used it may be useful to adjust its pH value to 6 or below, preferably 5 or below, and more preferably 4 or below. The concentration of the polysaccharide in a solution used for coating the nano- and/or microparticles preferably ranges from 0.1 to 10 g/l, preferably 1 to 10 g/l as the amount of solute in relation to the total volume of the solution. The coating typically contains 80 % by weight or more, preferably 90 % by weight or more, of the polysaccharide. It is particularly preferred that the coating consists of the polysaccharide.

Preferred polysaccharides for coating the nano- and/or microparticles are selected from chitosan, alginate, pectin, inulin, guar gum, dextran, chondrotin sulfate, hyaluronic acid and combinations of two or more thereof. In accordance with a particularly preferred embodiment, the polysaccharide coated onto the nano- and/or microparticles is adapted to the polymer contained in the matrix provided by the particles. Thus, for example, a polysaccharide containing positively charged groups, such as chitosan, is preferably coated onto nano- and/or microparticles containing a polymer with negatively charged groups, especially a copolymer of methacrylic acid defined as polymer (ii) above. As a further example, a polysaccharide containing negatively charged groups, such as alginate, is preferably coated onto nano- and/or microparticles containing a polymer with positively charged groups, especially a copolymer with units containing a trialkylammonium group as encompassed by the polymer (iii) defined above. Most preferred as polysaccharides for coating are chitosan and alginate, in particular chitosan.

The nano- and/or microparticles containing the therapeutic agent can be conveniently stored e.g. as dry powders.

In accordance with a further aspect, the present invention thus provides a pharmaceutical formulation (also referred to as a medicament) for oral administration comprising nano- and/or microparticles, which particles contain one or more therapeutic agents dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, which pharmaceutical formulation is obtainable by the process as claimed. It will be understood that the disclosure provided above with respect to the process of the invention and all preferred aspects thereof, e.g. with respect to particle sizes and size distributions, therapeutic agents, matrix components and in particular the polymers (i) to (iii), also applies with regard to the pharmaceutical formulation containing nano- and/or microparticles obtainable by the process.

As explained above, these formulations are generally adapted for oral administration to a patient, in particular a human patient. They are particularly suitable to achieve delivery of the therapeutic agent to and absorption of the therapeutic agent in the colon.

Optionally, a pharmaceutical formulation in accordance with the invention comprises in addition to the nano- and/or microparticles one or more pharmaceutically acceptable excipients. Exemplary pharmaceutically acceptable excipients that may be used in the formulation of the pharmaceutical compositions are selected from carriers, vehicles, diluents, solvents, such as monohydric alcohols, including ethanol or isopropanol, and polyhydric alcohols such as glycols and edible oils such as soybean oil, coconut oil, olive oil, safflower oil cottonseed oil, oily esters such as ethyl oleate, isopropyl myristate; binders, adjuvants, solubilizers, thickening agents, stabilizers, disintegrants, glidants, lubricating agents, buffering agents, emulsifiers, wetting agents, suspending agents, sweetening agents, colourants, flavours, preservatives, antioxidants, processing agents, drug delivery modifiers and drug delivery enhancers such as calcium phosphate, magnesium stearate, talc, monosaccharides, disaccharides, starch, gelatine, cellulose, methylcellulose, sodium carboxymethyl cellulose, dextrose, hydroxypropyl-β-cyclodextrin, polyvinylpyrrolidone, low melting waxes, or ion exchange resins. Other suitable pharmaceutically acceptable excipients are described in Remington's Pharmaceutical Sciences, 15th Ed., Mack Publishing Co., New Jersey (1991).

As will be understood, the pharmaceutical formulation in accordance with the invention is for use as a medicament or for use in the therapeutic or prophylactic treatment of the human or animal body, in particular the human body. According to the invention, the formulation is to be administered orally, in particular perorally. Thus, the pharmaceutical formulation in accordance with the invention is an oral formulation. In particular it takes a solid form which can be swallowed by the human or animal patient, preferably the form of a tablet or a capsule. Typically, the capsule is a capsule filled with the nano- and/or microparticles.

To that extent, the present invention also encompasses a process for the preparation of a pharmaceutical formulation, said pharmaceutical formulation comprising nano- and/or microparticles containing one or more therapeutic agents dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, wherein the process comprises a step of preparing the nano- and/or microparticles in accordance with the process of the invention as disclosed above. If necessary or desired, the process for the preparation of a pharmaceutical formulation or a medicament may include one or more subsequent steps of forming the final pharmaceutical formulation.

As will be understood, the pharmaceutical formulation prepared by this process is for use as a medicament or for use in the therapeutic or prophylactic treatment of the human or animal body, in particular the human body. According to the invention, the formulation is to be administered orally, in particular perorally. Thus, the pharmaceutical formulation or a preferred medicament prepared by the process of the invention is an oral formulation. In particular, it takes a solid form which can be swallowed by the human or animal patient, preferably the form of a tablet or a capsule.

Optionally, the process for the preparation of a pharmaceutical formulation or for the preparation of a medicament in accordance with the invention comprises, as additional steps after the preparation of the nano- and/or microparticles, one or more of the following:
(i) combining the nano- and/or microparticles with one or more pharmaceutically acceptable excipients;
(ii) providing the pharmaceutical formulation or the medicament in a solid form which can be swallowed by a human or animal patient, in particular a tablet or a capsule.

Preferred therapeutic agents contained in the pharmaceutical formulation are selected from raloxifen (i.e. [6-hydroxy-2-(4-hydroxyphenyl)-benzothiophen-3-yl]-[4-[2-(1-piperidyl)ethoxy]phenyl]-methanone), atorvastatin (i.e. ((*3R*,*5R*)-7-[2-(4-fluorophenyl)-3-phenyl-4-(phenylcarbamoyl)-5-propan-2-ylpyrrol-1-yl]-3,5-dihydroxyheptanoic acid), rosuvastatin (i.e. (3*R*,5*S*,6*E*)-7-[4-(4-fluorophenyl)-2-(*N*-methylmethanesulfonamido)-6-(propan-2-yl)pyrimidin-5-yl]-3,5-dihydroxyhept-6-enoic acid), quetiapine (i.e. (2-(2-(4-dibenzo[b,*f*][1,4]thiazepine-11-yl-1-piperazinyl)ethoxy)ethanol), duloxetine (i.e. ((+)-(*S*)-*N-*Methyl-3-(naphthalen-1-yloxy)-3-(thiophen-2-yl)propan-1-amine) and asenapine (i.e. (3a*RS*,12b*RS*)-*rel*-5-Chloro-2,3,3a,12b-tetrahydro-2-methyl-1*H*-dibenz[2,3:6,7]oxepino[4,5-c]pyrrole), including pharmaceutically acceptable salt forms of these agents.

Thus, the invention further provides the following preferred pharmaceutical formulations which are made available by the process in accordance with the invention.

A pharmaceutical formulation comprising nano- and/or microparticles, which particles contain raloxifen or a pharmaceutically acceptable salt thereof dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, and optionally containing a polysaccharide coating. In a particularly preferred embodiment, the pharmaceutical formulation is for use in the treatment or prevention of osteoporosis or breast cancer, and is to be administered orally. The therapeutic agent is delivered into the colon and systemically absorbed from the colon.

A pharmaceutical formulation comprising nano- and/or microparticles, which particles contain atorvastatin or a pharmaceutically acceptable salt thereof dispersed in non-crystalline form in a matrix containing one or more polymers selected from cellulose ethers, cellulose esters, copolymers of methacrylic acid with one or more (mcth)acrylic acid esters, copolymers of two or more (meth)acrylic acid esters and mixtures thereof, and optionally containing a polysaccharide coating. In a particularly preferred embodiment, the pharmaceutical formulation is for use in the treatment or prevention of a high blood cholesterol level, of dyslipidemia or of a cardiovascular disease, and is to be administered orally. The therapeutic agent is delivered into the colon and systemically absorbed from the colon.

A pharmaceutical formulation comprising nano- and/or microparticles, which particles contain rosuvastatin or a pharmaceutically acceptable salt thereof dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, and optionally containing a polysaccharide coating. In a particularly preferred embodiment, the pharmaceutical formulation is for use in the treatment or prevention of high blood cholesterol levels, of dyslipidemia or of a cardiovascular disease, and is to be administered orally. The therapeutic agent is delivered into the colon and systemically absorbed from the colon.

A pharmaceutical formulation comprising nano- and/or microparticles, which particles contain quetiapine or a pharmaceutically acceptable salt thereof dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, and optionally containing a polysaccharide coating. In a particularly preferred embodiment, the pharmaceutical formulation is for use in the treatment or prevention of schizophrenia, of a bipolar disorder or a depressive disorder, and is to be administered orally. The therapeutic agent is delivered into the colon and systemically absorbed from the colon.

A pharmaceutical formulation comprising nano- and/or microparticles, which particles contain asenapine or a pharmaceutically acceptable salt thereof dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, and optionally containing a polysaccharide coating. In a particularly preferred embodiment, the pharmaceutical formulation is for use in the treatment or prevention of schizophrenia, of a bipolar disorder or a depressive disorder, and is to be administered orally. The therapeutic agent is delivered into the colon and systemically absorbed from the colon.

A pharmaceutical formulation comprising nano- and/or microparticles, which particles contain aripiprazole or a pharmaceutically acceptable salt thereof dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, and optionally containing a polysaccharide coating. In a particularly preferred embodiment, the pharmaceutical formulation is for use in the treatment or prevention of schizophrenia, of a bipolar disorder or a depressive disorder, and is to be administered orally. The therapeutic agent is delivered into the colon and systemically absorbed from the colon.

A pharmaceutical formulation comprising nano- and/or microparticles, which particles contain duloxetine or a pharmaceutically acceptable salt thereof dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, and optionally containing a polysaccharide coating. In a particularly preferred embodiment, the pharmaceutical formulation is for use in the treatment or prevention of an anxiety disorder, of a depressive disorder, or of stress urinary incontinence, and is to be administered orally. The therapeutic agent is delivered into the colon and systemically absorbed from the colon.

### Examples

The following examples illustrate the invention, but it will be understood that the invention as defined above and in the annexed claims is not restricted to the embodiments specifically shown in these examples.

In the context of this application, powder X-ray diffraction was performed with a Philips PW 1830 generator equipped with a Philips PW 1820 vertical powder goniometer and a Philips PW 1710 control unit.

In vitro release profiles were determined as a prognostic tool for oral formulations according to the guidelines (FDA, Extended Release Oral Dosage Forms: Development, Evaluation, and Application of In Vitro/In VivoCorrelations, Center for Drug Evaluation and Research (CDER), 1997; FDA, SUPAC-MR: Modified Release Solid Oral Dosage Forms, Center for Drug Evaluation and Research (CDER), 1997). The method for the measurement of the in-vitro release profile is described below in detail.

All reagents are of analytical grade and were purchased form Sigma-Aldrich (Germany). Raloxifene Hydrochloride CRS (Y0001134 Batch 1.0) was purchased from EDQM (France). The in-vitro release method and buffer composition is following the setup conditions described in the literature (Dressmann J.B., Amidon G.L., Reppas C., Shah V.P., Dissolution testing as a prognostic tool for oral drug absorption: immediate release dosage forms, Pharmaceutical Research, 15(1), 1998; Fotaki N., Vertzoni M., Biorelevant Dissolution Methods an Their Applications in In Vitro-In Vivo Correlations for Oral Formulations, The Open Drug Delivery Journal, 4, 2010). The buffer systems were prepared without enzymes or natural surfactants, only with sodium lauryl sulphate as a synthetic surfactant. The composition of the dissolution media is described in Table 12. All reagents were added to deionised water (purified with MILLI-Q Gradient water system, Merck-Millipore) and stirred at room temperature to achieve a clear solution. The pH-Value of the media was controlled and adjusted with diluted hydrochloric acid or sodium hydroxide solution.

**Tab. 12: Composition of release media for in vitro testing**

| Medium | Composition | |
|---|---|---|
| Simulated Gastric Fluid, pH 1.2 (**SGF pH 1.2**) | Hydrochloric acid (36%) | 8.5 mL |
| | Sodium lauryl sulphate | 2.5 g |
| | NaCl | 2 g |

| | deionized water to | 1000 mL |
|---|---|---|
| Simulated Intestinal Fluid, pH 4.5 (**SIF pH 4.5**) | Glacial acetic acid | 8.27 mL |
| | Sodium lauryl sulphate | 0.5 g |
| | NaCl | 11.1 g |
| | deionized water to | 1000 mL |
| Simulated Intestinal Fluid, pH 6.8 (**SIF pH 6.8**) | NaH₂PO₄ ^{∗} 1 H₂O | 4 g |
| | Sodium lauryl sulphate | 1.44 g |
| | NaCl | 12.86 g |
| | deionized water to | 1000 mL |

Mesurement of the release profile was performed via a variation of the procedure of monograph 2375 published in the European Pharmacopoeia Supplement 6.6, using a USP 2 paddle apparatus (AT7 smart, Sotax, and Swiss) according to the requirements of the USP 26 / Eur.Pharm 6.0 (2.9.3). Drug containing formulations (50 ± 0.5 mg) were transferred into the vessels and the vessels were filled with 900 mL of tempered medium. The medium was stirred continuously by the paddle blades at 50 rpm and the temperature of the water bath was maintained at 37 ± 0.5°C. Manual sampling was conducted through a syringe filter (regenerated cellulose) fitted to a 2 mL-syringe. The sample volume was replenished with the same volume of fresh medium. The fresh medium was back-injected through the syringe filter so retained particles were washed back into the dissolution vessel. The amount of drug released at each sampling time was measured by RP-HPLC analysis.

### Example 1

2.0 g of the polymer Eudragit S100 is dissolved in 15 ml of a mixture of ethyl acetate and dimethylsulfoxide (DMSO) (60/40 (v/v)) and the solution is transferred to a double-walled glass vessel (inside height 16.0 cm, inside diameter 3.4 cm). 1.5 ml of DMSO solution containing 500 mg raloxifene hydrochloride is dispersed by means of a mechanical agitator (Dispermat FT, VMA-Getzmann GmbH, Germany, equipped with a 2.5 cm dissolver disc) in the polymer solution for 10 minutes at 2500 rpm and 6 minutes at 3000 rpm at room temperature.

50 mL PVA solution (0.5% (w/v)) in 100 mmol acetate buffer pH 4.0 containing 25.0 g sucrose is then added as continuous phase during agitation at 3000 rpm. Agitation is continued for about 60 seconds. The micro particle suspension is transferred to a beaker and 100 ml PVA solution (1% (w/v)) in 100 mmol acetate buffer pH.4.0 containing 50.0 g sucrose is added. The suspension of nano- and micro particles is agitated with a magnetic stirrer. After 30 min the nano- and micro particles are separated by centrifugation. Particles are reconstituted in a 1 L two-necked flask by addition of 100 ml PVA solution (1% (w/v)) in 100 mmol acetate buffer pH.4.0 containing 50.0 g sucrose. The solvent ethyl acetate is eliminated by applying vacuum. DMSO is removed by extraction. After 3 hours 50 mL PVA solution (1% (w/v)) in 100 mmol acetate buffer pH 4.0 is added and evaporation is continued for another 60 min. Nano- and micro particles are washed with 1 L 20 mm acetate buffer pH 4.0 and concentrated by centrifugation or filtration to the desired volume. Finally the nano- and micro particles are coated with 400 ml 1% chitosan solution pH 4.0, for two hour, filtered, washed and lyophilized. The lyophilisate, resuspended in appropriate solution with appropriate pH, contains nano- or micro particles.

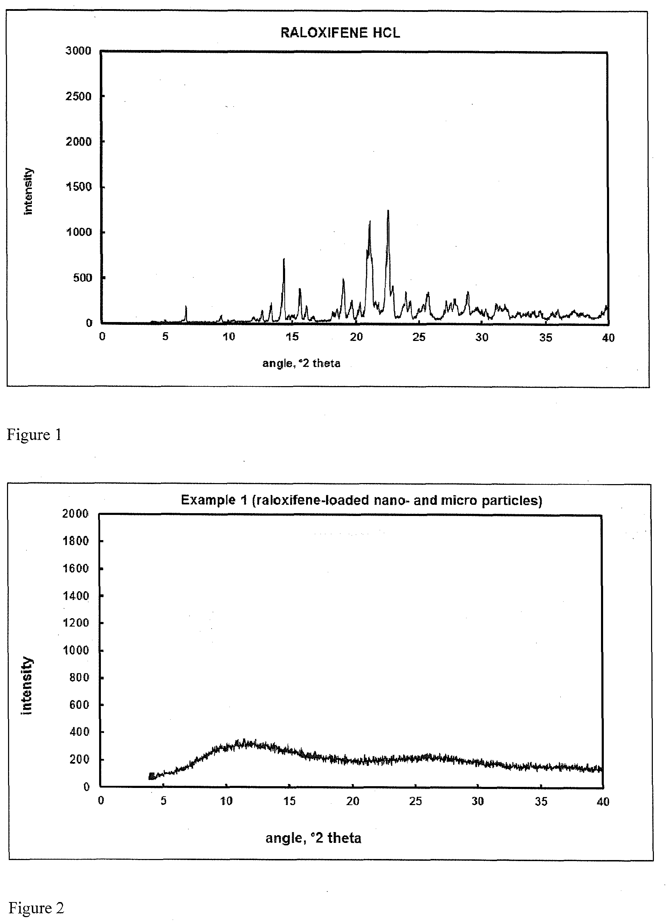

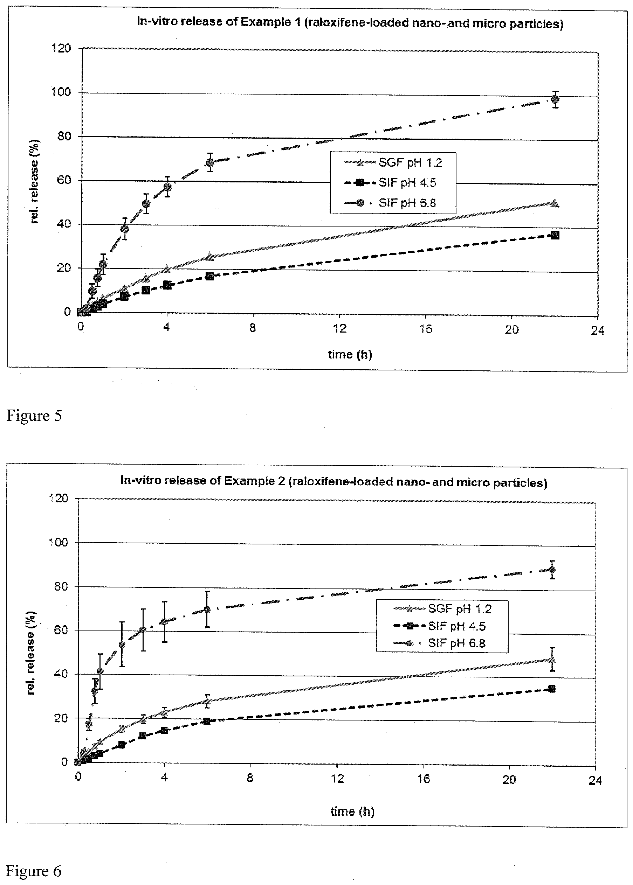

The in-vitro release profile determined for the particles of example 1 is shown in Figure 5. Powder X-ray diffraction was used to determine the structure of the active agent in the manufactured formulation in figure 2. The X-ray diffractogramm of raloxifene as reference is shown in figure 1.

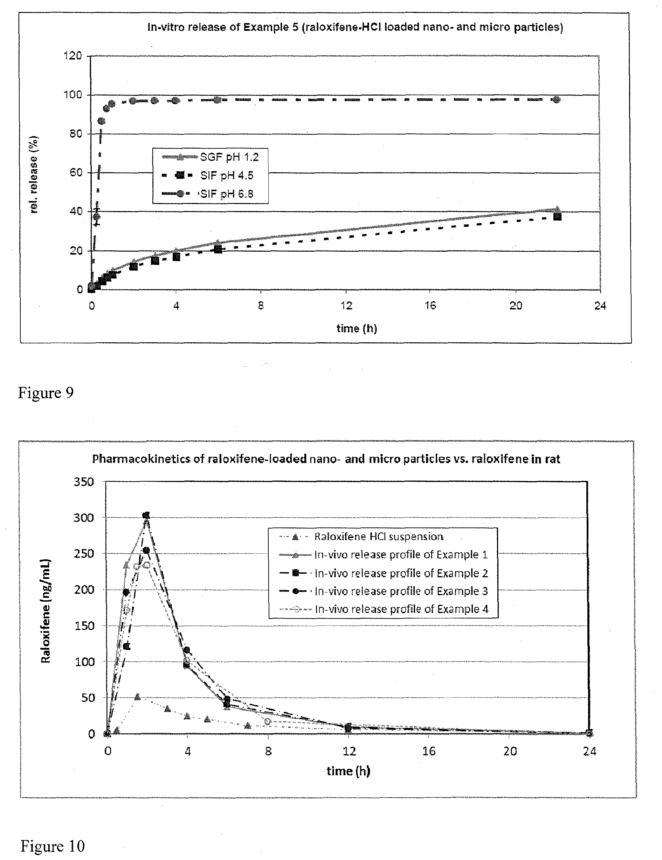

Pharmacokinetic studies were performed in rat. The particles of example 1 were administered orally to four rats at a dose of 10 mg/kg body weight. Figure 10 exhibits the in-vivo pharmacokinetics of the particles of example 1.

### Example 2

1.0 g of the polymer Eudragit S100 and 1.0 g of polymer Eudragit FS 30 D are dissolved in 15 ml of a mixture of ethyl acetate and dimethylsulfoxide (DMSO) (60/40 (v/v)) and the solution is transferred to a double-walled glass vessel (inside height 16.0 cm, inside diameter 3.4 cm). 1.0 ml DMSO solution containing 350 mg raloxifene hydrochloride is dispersed by means of a mechanical agitator (Dispermat FT, VMA-Getzmann GmbH, Germany, equipped with a 2.5 cm dissolver disc) in the polymer solution for 10 minutes at 3000 rpm at room temperature.

50 mL PVA solution (1% (w/v)) in 100 mmol acetate buffer pH. 4.0 containing 12.5 g sucrose is added as continuous phase during agitation at 3000 rpm. After about 60 seconds of agitation, the suspension of nano- and micro particles is transferred to a beaker and 50 mL PVA solution (1% (w/v)) in 100 mmol acetate buffer pH. 4.0 containing 12.5 g sucrose is added. The suspension of nano- and micro particles is agitated with a magnetic stirrer. After 60 min the nano- and micro particles are separated by centrifugation. Particles are reconstituted in a 1 L two-necked flask by addition of 80 ml PVA solution (1% (w/v)) in 100 mmol acetate buffer pH.4.0 containing 20.0 g sucrose. The solvent ethyl acetate is eliminated by applying a vacuum and DMSO is removed by extraction. After 45 min 50 mL PVA solution (1% (w/v)) in 100 mmol acetate buffer pH 4.0 containing 12.5 g sucrose is added and evaporation is continued for 150 min.

Nano- and micro particles are washed with 1 L 20 mm acetate buffer pH 4.0 and concentrated by centrifugation or filtration to the desired volume. Finally the nano- and micro particles are coated with 500 ml 10 mmol acetate buffer pH 4.0 containing 5g chitosan for 2 hours, filtered, washed and lyophilized. The lyophilisate, resuspended in appropriate solution with appropriate pH, contains nano or micro particles.

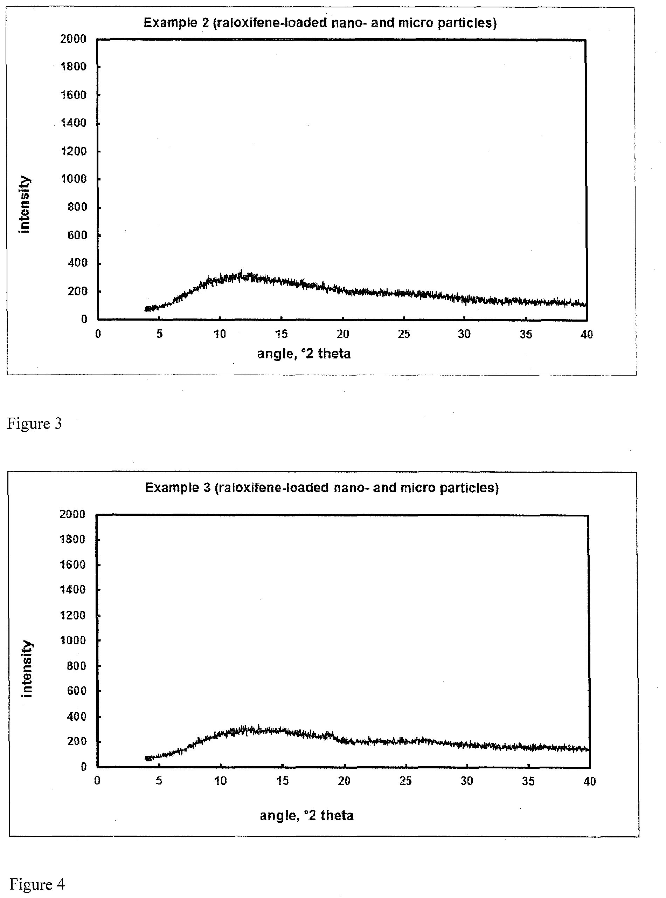

The in-vitro release profile of Example 2 is shown in Figure 6. Powder X-ray diffraction was used to determine the crystalline structure of the active agent in the manufactured formulation. Figure3 exhibits the X-ray diffractogramm of Example 2. Pharmacokinetic studies were performed in rat. Example 2 was administered orally to four rats at a dose of 10 mg/kg body weight. Figure 10 shows the in-vivo pharmacokinetics of example 2.

### Example 3

750 mg g of the polymer Eudragit S100 is dissolved in 10 ml of a mixture of ethyl acetate and dimethylsulfoxide (DMSO) (60/40 (v/v)) and the solution is transferred to a double-walled glass vessel (inside height 16.0 cm, inside diameter 3.4 cm). 1.0 ml DMSO solution containing 350 mg raloxifene hydrochloride is dispersed by means of a mechanical agitator (Dispermat FT, VMA-Getzmann GmbH, Germany, equipped with a 2.5 cm dissolver disc) in the polymer solution for 10 minutes at 3000 rpm at room temperature. Additionally, 250 mg of the polymer ethyl cellulose in 10 ml of a mixture of ethyl acetate and DMSO (60/40 (v/v)) is added and then dispersed by means of a mechanical agitator (Dispermat FT, VMA-Getzmann GmbH, Germany, equipped with a 2.5 cm dissolver disc) in the polymer solution for another 10 minutes at 3000rpm.

50 mL PVA solution (1% (w/v)) in 100 mmol acetate buffer pH. 4.0 containing 12.5 g sucrose is added as continuous phase during agitation at 3000 rpm. After about 60 seconds of agitation, the suspension of nano- and micro particles is transferred to a beaker and 100 mL PVA solution (1% (w/v)) in 100 mmol acetate buffer pH. 4.0 containing 25 g sucrose is added. The suspension of nano- and micro particles is agitated with a magnetic stirrer. After 15 min 100 mL PVA solution (1% (w/v)) in 100 mmol acetate buffer pH 4.0 containing 25 g sucrose is added and after another 15 min the nano- and micro particles are separated by centrifugation. Particles are re-constituted in a 1 L two-necked flask by addition of 100 ml PVA solution (1% (w/v)) in 100 mmol acetate buffer pH.4.0 containing 25.0 g sucrose. The solvent ethyl acetate is eliminated by applying a vacuum and DMSO is removed by extraction. After 150 min 50 mL PVA solution (1% (w/v)) in 100 mmol acetate buffer pH 4.0 containing 12.5 g sucrose is added and evaporation is continued for 60 min.

Nano- and micro particles are washed with 1 L 20 mm acetate buffer pH 4.0 and concentrated by centrifugation or filtration to the desired volume. Finally the concentrated nano- and micro particle suspension is lyophilized. The lyophilisate, resuspended in appropriate solution with appropriate pH, contains nano or micro particles.

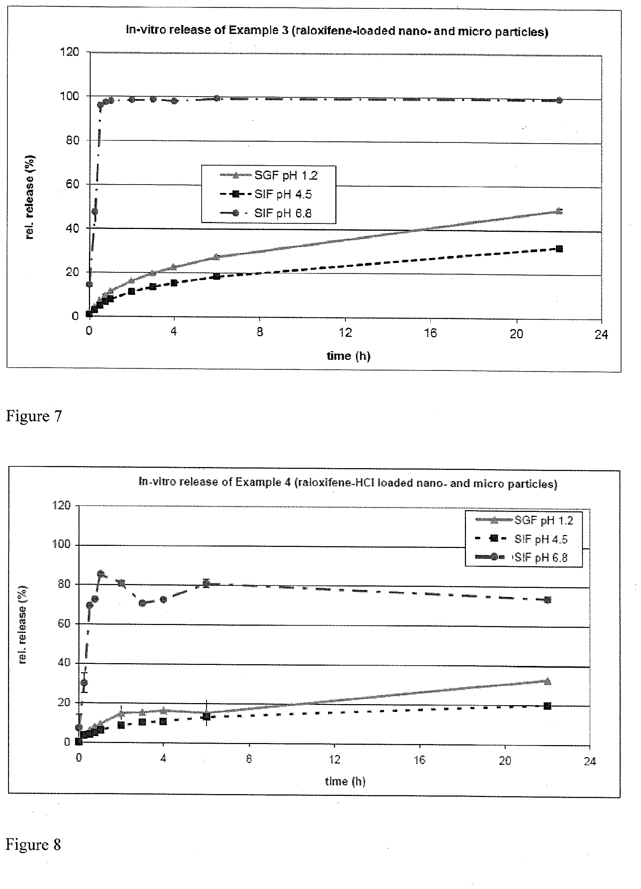

The in-vitro release profile of Example 3 is shown in Figure 7. Powder X-ray diffraction is used to determine the state of the active agent in the prepared formulation. Figure 4 exhibits the X-ray diffractogramm of Example 3. Pharmacokinetic studies were performed in rat. Example 3 was administered orally to four rats at a dose of 10 mg/kg body weight. Figure 10 exhibits the in-vivo pharmacokinetics of example 3.

### Example 4

1500 mg g of the polymer Eudragit S100 is dissolved in 10 ml of a mixture of ethyl acetate and dimethylsulfoxide (DMSO) (60/40 % (v/v)) and the solution is transferred to a double-walled glass vessel (inside height 16.0 cm, inside diameter 3.4 cm). 1.0 ml DMSO solution containing 350 mg raloxifene hydrochloride is dispersed by means of a mechanical agitator (Dispermat FT, VMA-Getzmann GmbH, Germany, equipped with a 2.5 cm dissolver disc) in the polymer solution for 10 minutes at 3000 rpm at room temperature. Additionally, 500 mg of the polymer ethyl cellulose in 10 ml of a mixture of ethyl acetate and DMSO (60/40 % (v/v)) is added and then dispersed by means of a mechanical agitator (Dispermat FT, VMA-Getzmann GmbH, Germany, equipped with a 2.5 cm dissolver disc) in the polymer solution for another 10 minutes at 3000rpm.

80 mL PVA solution (1% (w/v)) in 100 mmol acetate buffer pH. 4.0 containing 20.0g sucrose is added as continuous phase during agitation at 3000 rpm. After about 60 seconds of agitation, the suspension of nano- and micro particles is transferred to a beaker and 100 mL PVA solution (1% (w/v)) in 100 mmol acetate buffer pH. 4.0 containing 25 g sucrose is added. The suspension of nano- and micro particles is agitated with a magnetic stirrer. After 15 min 100 mL PVA solution (1% (w/v)) in 100 mmol acetate buffer pH 4.0 containing 25 g sucrose is added and after another 15 min the nano- and micro particles are separated by filtration. Particles are re-constituted in a 1 L two-necked flask by addition of 100 ml PVA solution (1% (w/v)) in 100 mmol acetate buffer pH.4.0 containing 25.0 g sucrose. The solvent ethyl acetate is eliminated by applying a vacuum and DMSO is removed by extraction. After 150 min 50 mL PVA solution (1% (w/v)) in 100 mmol acetate buffer pH 4.0 containing 12.5 g sucrose is added and evaporation is continued for 60 min. Nano- and micro particles are washed with 1 L 20 mm acetate buffer pH 4.0 and concentrated by centrifugation or filtration to the desired volume. Finally the concentrated nano- and micro particle suspension is lyophilized. The lyophilisate, resuspended in appropriate solution with appropriate pH, contains nano or micro particles.

Figure 8 shows the in-vitro release profile of example 4. Pharmacokinetic studies were performed in rat. Example 4 was administered orally to four rats at a dose of 10 mg/kg body weight. In-vivo pharmacokinetics of example 4 is shown in Figure 10.

### Example 5

3.0g of the polymer Eudragit RS100 is dissolved in 15 ml of a mixture of ethyl acetate and dimethylsulfoxide (DMSO) (60/40 % (v/v)) and the solution is transferred to a double-walled glass vessel (inside height 16.0 cm, inside diameter 3.4 cm). 1.5 ml DMSO solution containing 500 mg raloxifene hydrochloride is dispersed by means of a mechanical agitator (Dispermat FT, VMA-Getzmann GmbH, Germany, equipped with a 2.5 cm dissolver disc) in the polymer solution for 20 minutes at 3000 rpm at room temperature.

50 mL PVA solution (1% (w/v)) in 100 mmol citrate buffer pH. 6.5 containing 10.0 g sucrose is added as continuous phase during agitation at 3000 rpm. After about 60 seconds of agitation, the suspension of nano- and micro particles is transferred to a beaker and 100 mL PVA solution (1% (w/v)) in 100 mmol citrate buffer pH. 6.5 containing 20 g sucrose is added. The suspension of nano- and micro particles is agitated with a magnetic stirrer. After 15 min 100 mL PVA solution (1% (w/v)) in 100 mmol citrate buffer pH 6.5 containing 20 g sucrose is added and after another 15 min the nano- and micro particles are separated by filtration.

Particles are re-constituted in a 1 L two-necked flask by addition of 100 ml PVA solution (1% (w/v)) in 100 mmol citrate buffer pH.6.5 containing 20.0 g sucrose. The solvent ethyl acetate is eliminated by applying a vacuum and DMSO is removed by extraction. After 150 min 50 mL PVA solution (1% (w/v)) in 100 mmol citrate buffer pH 6.5 containing 10.0 g sucrose is added and evaporation is continued for 60 min.

Nano- and micro particles are washed with 1 L 20 mm citrate buffer pH 6.5 and concentrated by centrifugation or filtration to the desired volume. Finally the concentrated nano- and micro particles are coated with 700 ml 1% sodium alginate for 2 hours, filtered and lyophilized. The lyophilisate, resuspended in appropriate solution with appropriate pH, contains nano or micro particles. The in vitro release profile of the example 5 is shown in Figure 9.

### Example 6

2.0 g of the polymer Eudragit RS100 is dissolved in 10 ml ethyl acetate and the solution is transferred to a double-walled glass vessel (inside height 16.0 cm, inside diameter 3.4 cm). 1.0 ml NMP solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 20 minutes at 3000 rpm at room temperature.

50 mL PVA solution (0.5% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1% (w/v)) in 100 mmol acetate buffer pH. 4.0 containing 25 g sucrose is added. The suspension of nano- and micro particles is agitated with a magnetic stirrer. After 20 min the nano- and micro particles are separated by centrifugation. Particles are re-constituted in a 1 L two-necked flask by addition of 100 ml PVA solution (1% (w/v)) in 100 mmol acetate buffer pH.4.0 containing 25.0 g sucrose. The solvent ethyl acetate is eliminated by applying vacuum and NMP is removed by extraction. After 2 hours, the suspension is washed with 1L 20 mmol acetate buffer pH 4.0 and concentrated by centrifugation or filtration to the desired volume. Finally the concentrated nano- and micro particle suspension is lyophilized. The lyophilizate, resuspended in appropriate solution with appropriate pH, contains nano- or micro particles.

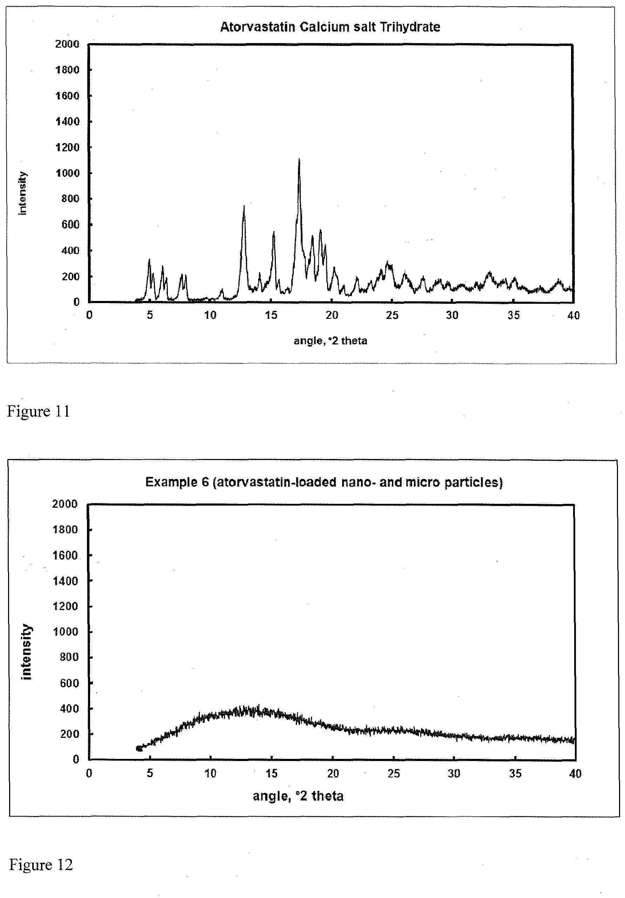

Powder X-ray diffraction was used to determine the crystalline structure of the active agent in the manufactured formulation. As a reference the X-ray diffractogramm of atorvastatin is shown in Figure 11. Figure 12 exhibits the X-ray diffractogramm of Example 6.

### Example 7

2.0 g of the polymer Eudragit RS100 is dissolved in 10 ml ethyl acetate and the solution is transferred to a double-walled glass vessel (inside height 16.0 cm, inside diameter 3.4 cm). 1.0 ml DMSO solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 20 minutes at 3000 rpm at room temperature.

50 mL PVA solution (0.5% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1% (w/v)) in 100 mmol acetate buffer pH. 4.0 containing 25 g sucrose is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1% (w/v)) in 100 mmol acetate buffer pH.4.0 containing 25.0 g sucrose. The solvent ethyl acetate is eliminated by applying vacuum and DMSO is removed by extraction. After 3.5 hours the suspension is washed with 1 L 20 mmol acetate buffer pH 34.0 containing 0.25% PVA (w/v) and concentrated by centrifugation or filtration to the desired volume. Finally the concentrated nano- and micro particle suspension is lyophilized. The lyophilizate, resuspended in appropriate solution with appropriate pH, contains nano- or micro particles.

### Example 8

2.0 g of the polymer Eudragit RS100 is dissolved in 10 ml of ethyl acetate and the solution is transferred to a double-walled glass vessel (inside height 16.0 cm, inside diameter 3.4 cm). 1.0 ml of glycofurol solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 30 min at 3000 rpm at room temperature.

50 mL PVA solution (0.5% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (0.5% (w/v)) in 100 mmol acetate buffer pH. 4.0 containing 25 g sucrose is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1% (w/v)) in 100 mmol acetate buffer pH.4.0 containing 25.0 g sucrose. The solvent ethyl acetate is eliminated at room temperature by applying a vacuum and glycofurol is removed by extraction. After 3 hours the suspension is washed with 1 L 20 mmol acetate buffer pH 3.6 containing 0.25% Poloxamer 188 (w/v) and concentrated by centrifugation or filtration to the desired volume. Finally the concentrated nano- and micro particles are lyophilized. The lyophilizate, resuspended in appropriate solution with appropriate pH, contains nano- or micro particles.

### Example 9

2.0 g of the polymer Eudragit RS100 is dissolved in 10 ml of ethyl acetate and the solution is transferred to a double-walled glass vessel (inside height 16.0 cm, inside diameter 3.4 cm). 1.0 ml of THF solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 20 min at 3000 rpm at room temperature.

50 mL PVA solution (0.5% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a 1 L two-necked flask and 100 mL PVA solution (1% (w/v)) in 100 mmol acetate buffer pH. 4.0 containing 25 g sucrose is added. The solvents ethyl acetate and THF are eliminated at room temperature by applying a vacuum. The suspension of nano- and micro particles is agitated with a magnetic stirrer. After 3 hours the suspension is washed with 1 L 20 mmol acetate buffer pH 3.6 containing 0.25% Poloxamer 188 (w/v) and concentrated by centrifugation or filtration to the desired volume. Finally the concentrated nano- and micro particles are lyophilized. The lyophilizate, resuspended in appropriate solution with appropriate pH, contains nano- or micro particles.

### Example 10

2.0 g of the polymer Eudragit RS100 is dissolved in 10 ml of ethyl acetate and the solution is transferred to a double-walled glass vessel (inside height 16.0 cm, inside diameter 3.4 cm). 1.0 ml of acetone solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 20 min at 3000 rpm at room temperature.

50 mL PVA solution (0.5% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a 1 L two-necked flask and 100 mL PVA solution (1% (w/v)) in 100 mmol acetate buffer pH. 4.0 containing 25 g sucrose is added. The solvents ethyl acetate and acetone are eliminated at room temperature by applying a vacuum. The suspension of nano- and micro particles is agitated with a magnetic stirrer. After 3 hours the suspension is washed with 1 L 20 mmol acetate buffer pH 4.0 and concentrated by centrifugation or filtration to the desired volume. Finally the concentrated nano- and micro particles are lyophilized. The lyophilizate, resuspended in appropriate solution with appropriate pH, contains nano- or micro particles.

### Example 11

2.0 g of the polymer Eudragit L100-55 is dissolved in 15 ml of a mixture of ethyl acetate and glycofurol 60/40 (v/v) and the solution is transferred to a double-walled glass vessel (inside height 16.0 cm, inside diameter 3.4 cm). 1.0 ml of glycofurol solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 5min at 2000 rpm at room temperature.

50 mL PVA solution (0.5% (w/v)) in 100 mmol acetate buffer pH 4.0 containing 12.5 g sucrose is then added as continuous phase during agitation at 2000 rpm. After about 60 seconds of agitation, the suspension of nano- and micro particles is transferred to a beaker and 100 mL PVA solution (0.5% (w/v)) in 100 mmol acetate buffer pH. 4.0 containing 25 g sucrose is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1% (w/v)) in 100 mmol acetate buffer pH 4.0 containing 25.0 g sucrose. The solvent ethyl acetate is eliminated at room temperature by applying a vacuum and glycofurol is removed by extraction. After 2 h 50 mL PVA solution (1% (w/v)) in 100 mmol acetate buffer pH 4.0 containing 12.5.0 g sucrose is added and vacuum is applied for another 60 min. Nano- and micro particles are washed with 2 L 20 mmol acetate buffer pH 4.0 and the suspension is concentrated by centrifugation or filtration to the desired volume. Finally the nano- and micro particles are coated with 200 ml 10 mmol acetate buffer pH 3.6 containing 2g chitosan for one hour, filtered, washed and lyophilized. The lyophilisate, resuspended in appropriate solution with appropriate pH, contains nano or micro particles.

### Example 12

2.0 g of the polymer Eudragit L100-55 is dissolved in 15 ml of a mixture of ethyl acetate and glycofurol 60/40 (v/v) and the solution is transferred to a double-walled glass vessel (inside height 16.0 cm, inside diameter 3.4 cm). 1.0 ml of glycofurol solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 5min at 2000 rpm at room temperature.

50 mL PVA solution (0.5% (w/v)) in 100 mmol acetate buffer pH 4.0 containing 2.5 g NaCl is then added as continuous phase during agitation at 2000 rpm. After about 60 seconds of agitation, the suspension of nano- and micro particles is transferred to a beaker and 100 mL PVA solution (0.5% (w/v)) in 100 mmol acetate buffer pH. 4.0 containing 5 g NaCl is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1% (w/v)) in 100 mmol acetate buffer pH.4.0 containing 5.0 g NaCl. The solvent ethyl acetate is eliminated at room temperature by applying a vacuum and glycofurol is removed by extraction. After 2 h 50 mL PVA solution (1% (w/v)) in 100 mmol acetate buffer pH 4.0 containing 2.5 g NaCl is added and vacuum is applied for another 60 min. Nano- and micro particles are washed with 2 L 20 mmol acetate buffer pH 4.0 and the suspension is concentrated by centrifugation or filtration to the desired volume. Finally the nano- and micro particles are lyophilised. The lyophilisate, resuspended in appropriate solution with appropriate pH, contains nano- or micro particles.

### Example 13

2.0 g of the polymer Eudragit L100 is dissolved in 15 ml of a mixture of ethyl acetate and glycofurol 60/40 (v/v) and the solution is transferred to a double-walled glass vessel (inside height 16.0 cm, inside diameter 3.4 cm). 1.0 ml of glycofurol solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 15min at 3000 rpm at room temperature.

50 mL PVA solution (0.5% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (0.5% (w/v)) in 100 mmol acetate buffer pH 4.0 containing 25 g sucrose is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1% (w/v)) in 100 mmol acetate buffer pH 4.0 containing 25.0 g sucrose. The solvent ethyl acetate is eliminated at room temperature by applying a vacuum and glycofurol is removed by extraction. After 2 h 50 mL PVA solution (1% (w/v)) in 100 mmol acetate buffer pH 4.0 containing 12.5 g sucrose is added and vacuum is applied for another 60 min. Nano- and micro particles are washed with 2 L 10 mmol acetate buffer pH 4.0 and the suspension is concentrated by centrifugation or filtration to the desired volume. Finally the nano- and micro particles are lyophilised. The lyophilisate, resuspended in appropriate solution with appropriate pH, contains nano- or micro particles.

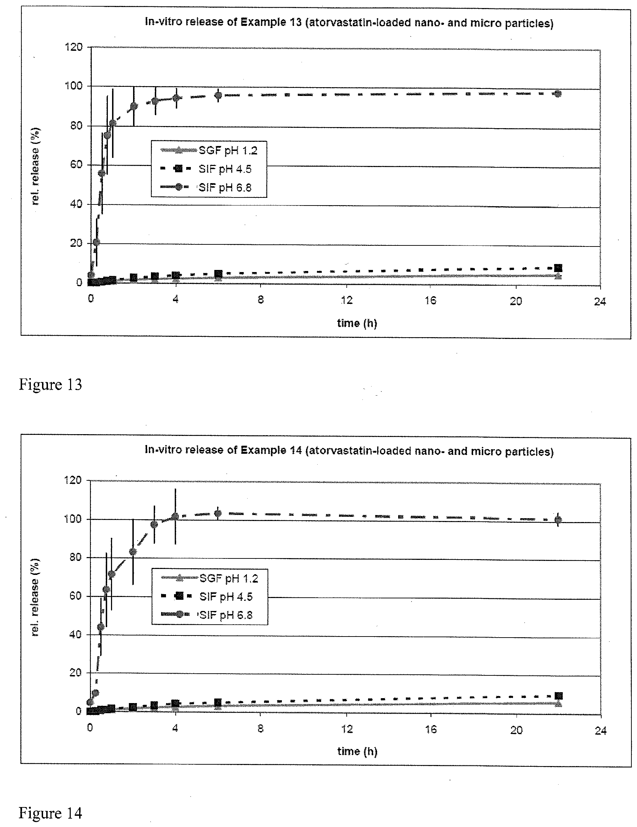

Figure 13 shows the in-vitro release profile of example 13.

### Example 14

2.0 g of the polymer Eudragit S100 is dissolved in 15 ml of a mixture of ethyl acetate and glycofurol 60/40 (v/v) and the solution is transferred to a double-walled glass vessel (inside height 16.0 cm, inside diameter 3.4 cm). 1.0 ml of glycofurol solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 7 min at 2000 rpm and for 11min at 3000 rpm at room temperature.

50 mL PVA solution (0.5% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1% (w/v)) in 100 mmol acetate buffer pH. 4.0 containing 25 g sucrose is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1% (w/v)) in 100 mmol acetate buffer pH 4.0 containing 25.0 g sucrose. The solvent ethyl acetate is eliminated at room temperature by applying a vacuum and glycofurol is removed by extraction. After 3.5 h the nano- and micro particles are washed with 2 L 20 mm acetate buffer pH 3.8 and concentrated by centrifugation or filtration to the desired volume. Finally the nano- and micro particles are coated with 200 ml 1% chitosan solution pH 4.0, for 2 hours, filtered, washed and lyophilized. The lyophilisate, resuspended in appropriate solution with appropriate pH, contains nano or micro particles.

The in-vitro release profile of Example 14 is shown in Figure 14.

### Example 15

2.0 g of the polymer Eudragit S100 is dissolved in 15 ml of a mixture of ethyl acetate and glycofurol 60/40 (v/v) and the solution is transferred to a double-walled glass vessel (inside height 16.0 cm, inside diameter 3.4 cm). 1.0 ml of glycofurol solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 7 min at 2000 rpm and for 11min at 3000 rpm at room temperature.

50 mL PVA solution (0.5% (w/v)) in 100 mmol acetate buffer pH 4.0 containing 2.5 g NaCl is then added as continuous phase during agitation at 3000 rpm. After about 60 seconds of agitation, the suspension of nano- and micro particles is transferred to a beaker and 100 mL PVA solution (1% (w/v)) in 100 mmol acetate buffer pH. 4.0 containing 5 g NaCl is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1% (w/v)) in 100 mmol acetate buffer pH 4.0 containing 5.0 g NaCl. The solvent ethyl acetate is eliminated at room temperature by applying a vacuum and glycofurol is removed by extraction. After 3.5 h the nano- and micro particles are washed with 2 L 20 mm acetate buffer pH 3.8 and concentrated by centrifugation or filtration to the desired volume. Finally the nano- and micro particles are coated with 200 ml 1% chitosan solution pH 4.0, for one hour, filtered and lyophilized The lyophilisate, resuspended in appropriate solution with appropriate pH, contains nano or micro particles.

### Example 16

2.0 g of the polymer Ethylcellulose is dissolved in 15 ml of a mixture of ethyl acetate and glycofurol 60/40 (v/v) and the solution is transferred to a double-walled glass vessel (inside height 16.0 cm, inside diameter 3.4 cm). 1.0 ml of glycofurol solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 15min at 3000 rpm at room temperature.

50 mL PVA solution (0.5% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (0.5% (w/v)) in 100 mmol acetate buffer pH. 4.0 containing 25 g sucrose is added. The suspension of nano- and micro particles is agitated with a magnetic stirrer. 100 mL PVA solution (0.5% (w/v)) in 100 mmol acetate buffer pH. 4.0 containing 25 g sucrose is added after 15 min, and 250 mL 20 mmol acetate buffer pH 3.8 is added after 30 min, respectively. After 40 min the nano- and micro particles are separated by centrifugation. Particles are re-constituted in a 1 L two-necked flask by addition of 100 ml PVA solution (1% (w/v)) in 100 mmol acetate buffer pH 4.0 containing 25.0 g sucrose. The solvent ethyl acetate is eliminated at room temperature by applying a vacuum and glycofurol is removed by extraction. After 2 h 50 mL PVA solution (1% (w/v)) in 100 mmol acetate buffer pH 4.0 containing 12.5 g sucrose is added and vacuum is applied for another 60 min. The nano- and micro particles are washed with 2 L 20 mmol acetate buffer pH 4.0 and the suspension is concentrated by centrifugation or filtration to the desired volume. Finally the nano- and micro particles are lyophilised. The lyophilisate, resuspended in appropriate solution with appropriate pH, contains nano- or micro particles.

### Example 17

2.0 g of the polymer HPMC AS_HG is dissolved in 15 ml of a mixture of ethyl acetate and glycofurol 60/40 (v/v) and the solution is transferred to a double-walled glass vessel (inside height 16.0 cm, inside diameter 3.4 cm). 1.0 ml of glycofurol solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 15min at 3000 rpm at room temperature.

50 mL PVA solution (0.5% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (0.5% (w/v)) in 100 mmol acetate buffer pH. 4.0 containing 25 g sucrose is added. The suspension of nano- and micro particles is agitated with a magnetic stirrer. 100 mL PVA solution (0.5% (w/v)) in 100 mmol acetate buffer pH. 4.0 containing 25 g sucrose is added after 15 min, and 250 mL 20 mmol acetate buffer pH 3.8 is added after 30 min, respectively. After 40 min the nano- and micro particles are separated by centrifugation. Particles are re-constituted in a 1 L two-necked flask by addition of 100 ml PVA solution (1% (w/v)) in 100 mmol acetate buffer pH 4.0 containing 25.0 g sucrose. The solvent ethyl acetate is eliminated at room temperature by applying a vacuum and glycofurol is removed by extraction. After 2 h 50 mL PVA solution (1% (w/v)) in 100 mmol acetate buffer pH.4.0 containing 12.5 g sucrose is added and vacuum is applied for another 60 min. The nano- and micro particles are washed with 2 L 20 mmol acetate buffer pH 3.6 containing 0.5% Poloxamer 188. Finally the suspension is concentrated by centrifugation or filtration to the desired volume, and lyophilised. The lyophilisate, resuspended in appropriate solution with appropriate pH, contains nano- or micro particles.

### Example 18

2.0 g of the polymer CAP is dissolved in 15 ml of a mixture of ethyl acetate and glycofurol 60/40 (v/v) and the solution is transferred to a double-walled glass vessel (inside height 16.0 cm, inside diameter 3.4 cm). 1.0 ml of glycofurol solution containing 350 mg atorvastatin Ca salt trihydrate is dispersed by means of a mechanical agitator (Dispermat FT, VMA-Getzmann GmbH, Germany, equipped with a 2.5 cm dissolver disc) in the polymer solution for 10min at 3000 rpm at room temperature.

50 mL PVA solution (0.5% (w/v)) in 100 mmol succinate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (0.5% (w/v)) in 100 mmol succinate buffer pH. 4.0 containing 25 g sucrose is added. The suspension of nano- and micro particles is agitated with a magnetic stirrer. 100 mL PVA solution (0.5% (w/v)) in 100 mmol succinate buffer pH. 4.0 containing 25 g sucrose is added after 15 min, and 250 mL 20 mmol acetate buffer pH 3.6 is added after 30 min, respectively. After 40 min the nano- and micro particles are separated by centrifugation. Particles are re-constituted in a 1 L two-necked flask by addition of 100 ml PVA solution (1% (w/v)) in 100 mmol succinate buffer pH 4.0 containing 25.0 g sucrose. The solvent ethyl acetate is eliminated at room temperature by applying a vacuum and glycofurol is removed by extraction. After 2 h 50 mL PVA solution (1% (w/v)) in 100 mmol succinate buffer pH 4.0 containing 12.5 g sucrose is added and vacuum is applied for another 60 min. The nano- and micro particles are washed with 2 L 20 mmol acetate buffer pH 3.8 containing 0.5% Poloxamer 188 by centrifugation or filtration. Finally the suspension is concentrated by centrifugation or filtration to the desired volume, and lyophilised. The lyophilisate, resuspended in appropriate solution with appropriate pH, contains nano- or micro particles.

### Example 19

2.0 g of the polymer Eudragit S100 is dissolved in 15 ml of a mixture of isopropyl formate and glycofurol 60/40 (v/v) and the solution is transferred to a double-walled glass vessel (inside height 16.0 cm, inside diameter 3.4 cm). 1.0 mL DMSO solution containing 350 mg rosuvastatin Ca salt is dispersed by means of a mechanical agitator (Dispermat FT, VMA-Getzmann GmbH, Germany, equipped with a 2.5 cm dissolver disc) in the polymer solution for 15min at 3000 rpm at room temperature.

50 mL PVA solution (0.5% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (0.5% (w/v)) in 100 mmol acetate buffer pH. 4.0 containing 25 g sucrose is added. The suspension of nano- and micro particles is agitated with a magnetic stirrer. After 20 min the nano- and micro particles are separated by centrifugation. Particles are re-constituted in a 1 L two-necked flask by addition of 100 ml PVA solution (0.05% (w/v)) in 100 mmol acetate buffer pH 4.0 containing 25.0 g sucrose. The solvent isopropyl formate is eliminated at room temperature by applying a vacuum, and glycofurol is removed by extraction. After 90 min the nano- and micro particles are washed with 2 L 20 mm acetate buffer pH 3.8 and concentrated by centrifugation or filtration to the desired volume. Finally the nano- and micro particles are coated with 200 ml 1% chitosan solution pH 3.6 for one hour, filtered and lyophilized. The lyophilisate, resuspended in appropriate solution with appropriate pH, contains nano or micro particles.

### Example 20

2.0 g of the polymer ethylcellulose is dissolved in 15 ml of a mixture of ethyl acetate and glycofurol 60/40 (v/v) and the solution is transferred to a double-walled glass vessel (inside height 16.0 cm, inside diameter 3.4 cm). 1.0 ml of glycofurol solution containing 350 mg rosuvastatin Ca salt is dispersed by means of a mechanical agitator (Dispermat FT, VMA-Getzmann GmbH, Germany, equipped with a 2.5 cm dissolver disc) in the polymer solution for 15min at 3000 rpm at room temperature.

50 mL PVA solution (0.05% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (0.5% (w/v)) in 100 mmol acetate buffer pH 4.0 containing 25 g sucrose is added. The suspension of nano- and micro particles is agitated with a magnetic stirrer. 100 mL PVA solution (0.5% (w/v)) in 100 mmol acetate buffer pH. 4.0 containing 25 g sucrose is added after 15 min, and 250 mL 20 mmol acetate buffer pH 4.0 is added after 30 min, respectively. After 40 min the nano- and micro particles are separated by filtration. Particles are re-constituted in a 1 L two-necked flask by addition of 100 ml PVA solution (0.05% (w/v)) in 100 mmol acetate buffer pH4.0 containing 25.0 g sucrose. The solvent ethyl acetate is eliminated at room temperature by applying a vacuum and glycofurol is removed by extraction. After 2 h 50 mL PVA solution (0.05% (w/v)) in 100 mmol acetate buffer pH 4.0 containing 12.5 g sucrose is added and vacuum is applied for another 60 min. The nano- and micro particles are washed with 2 L 20 mmol acetate buffer pH 4.0 containing 0.5% Poloxamer 188 by centrifugation or filtration. Finally the suspension is concentrated by centrifugation or filtration to the desired volume, and lyophilised. The lyophilisate, resuspended in appropriate solution with appropriate pH, contains nano- or micro particles.

### Example 21

1.5g of the polymer Eudragit S100 is dissolved in 10 ml of a mixture of ethyl acetate and DMSO 60/40 (v/v) and polymer solution is transferred to a double-walled glass vessel (inside height 16.0 cm, inside diameter 3.4 cm). 1.5 ml of DMSO solution containing 350 mg rosuvastatin Ca salt is dispersed by means of a mechanical agitator (Dispermat FT, VMA-Getzmann GmbH, Germany, equipped with a 2.5 cm dissolver disc) in the polymer solution for 7 min at 3000 rpm at room temperature. Additionally, 500 mg of the polymer ethyl cellulose in 10 ml of a mixture of ethyl acetate and DMSO 60/40 (v/v) is added and then dispersed by means of a mechanical agitator (Dispermat FT, VMA-Getzmann GmbH, Germany, equipped with a 2.5 cm dissolver disc) in the polymer solution for another 8 minutes at 3000rpm.

50 mL PVA solution (1.0 % (w/v)) in 100 mmol acetate buffer pH 4.0 containing 20.0 g sucrose is then added as continuous phase during agitation at 3000 rpm. After about 60 seconds of agitation, the suspension of nano- and micro particles is transferred to a beaker and 100 mL PVA solution (1.0 % (w/v)) in 100 mmol acetate buffer pH 4.0 containing 25 g sucrose is added. The suspension of nano- and micro particles is agitated with a magnetic stirrer. 100 mL PVA solution (0.5% (w/v)) in 100 mmol acetate buffer pH 4.0 containing 25 g sucrose is added after 15 min, and 250 mL 20 mmol acetate buffer pH 4.0 is added after 30 min, respectively. After 40 min the nano- and micro particles are separated by filtration. Particles are re-constituted in a 1 L two-necked flask by addition of 100 ml PVA solution (1.0% (w/v)) in 100 mmol acetate buffer pH 4.0 containing 25.0 g sucrose. The solvent ethyl acetate is eliminated at room temperature by applying a vacuum and DMSO is removed by extraction. After 2 h 50 mL PVA solution (0.50% (w/v)) in 100 mmol acetate buffer pH 4.0 containing 12.5 g sucrose is added and vacuum is applied for another 60 min. The nano- and micro particles are washed with 2 L 20 mmol acetate buffer pH 4.0 containing 0.5% PVA centrifugation or filtration. Finally the suspension is concentrated by centrifugation or filtration to the desired volume, and lyophilised. The lyophilisate, resuspended in appropriate solution with appropriate pH, contains nano- or micro particles.

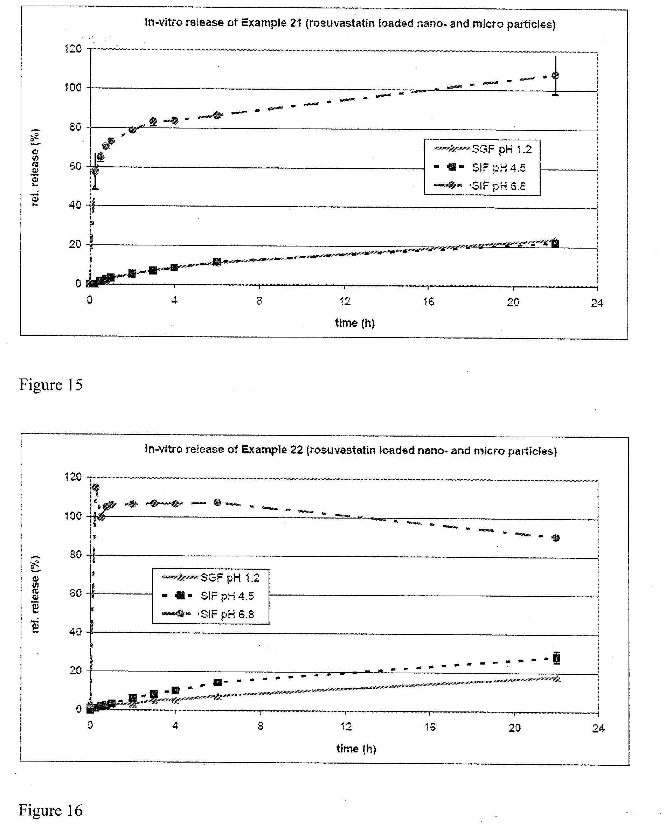

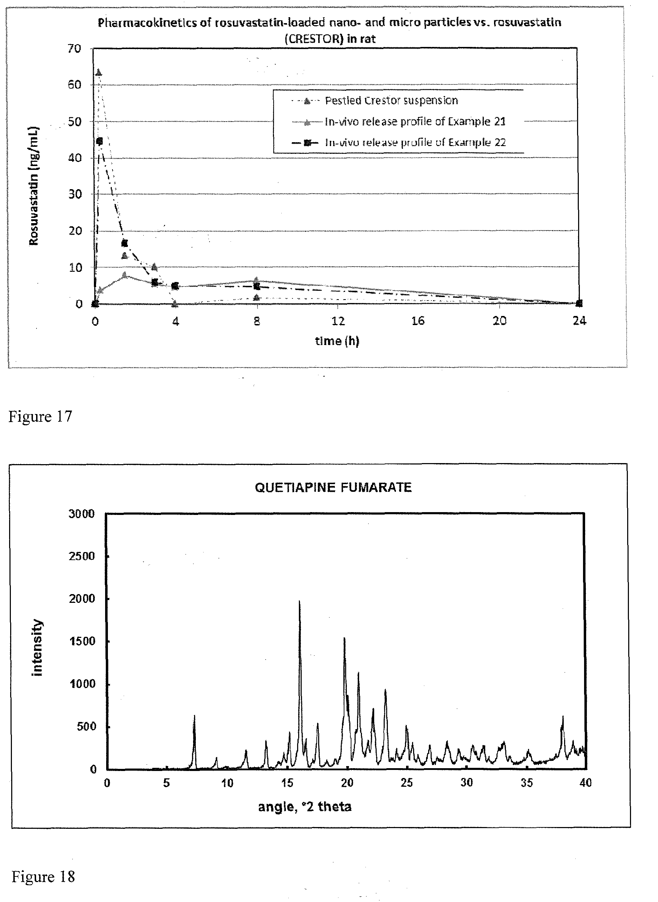

Figure 15 shows the *in-vitro* release profile of the particles of example 21. The particles of example 21 were administered orally to four rats at a dose of 10 mg/kg body weight. Figure 17 shows the *in-vivo* pharmacokinetics of example 21.

### Example 22

1.5g of the polymer Eudragit S100 and 500 mg HPMC AS-HG are dissolved in 15 ml of a mixture of ethyl acetate and DMSO 60/40 (v/v) and the solution is transferred to a double-walled glass vessel (inside height 16.0 cm, inside diameter 3.4 cm). 1.5 ml of DMSO solution containing 350 mg rosuvastatin Ca salt is dispersed by means of a mechanical agitator (Dispermat FT, VMA-Getzmann GmbH, Germany, equipped with a 2.5 cm dissolver disc) in the polymer solution for 10 min at 3000 rpm at room temperature.

80 mL PVA solution (1.0 % (w/v)) in 100 mmol acetate buffer pH 4.0 containing 20.0g sucrose is then added as continuous phase during agitation at 3000 rpm. After about 60 seconds of agitation, the suspension of nano- and micro particles is transferred to a beaker and 100 mL PVA solution (1.0 % ((w/v)) in 100 mmol acetate buffer pH 4.0 containing 25 g sucrose is added. The suspension of nano- and micro particles is agitated with a magnetic stirrer. 100 mL PVA solution (0.5% (w/v)) in 100 mmol acetate buffer pH 4.0 containing 25 g sucrose is added after 15 min, and 250 mL 20 mmol acetate buffer pH 4.0 is added after 30 min, respectively. After 40 min the nano- and micro particles are separated by filtration. Particles are re-constituted in a 1 L two-necked flask by addition of 100 ml PVA solution (1.0% (w/v)) in 100 mmol acetate buffer pH 4.0 containing 25.0 g sucrose. The solvent ethyl acetate is eliminated at room temperature by applying a vacuum and glycofurol is removed by extraction. After 2 h 50 mL PVA solution (1.0% (w/v)) in 100 mmol acetate buffer pH 4.0 containing 12.5 g sucrose is added and vacuum is applied for another 60 min. The nano- and micro particles are washed with 2 L 20 mmol acetate buffer pH 4.0 containing 0.5% PVA centrifugation or filtration. Finally the suspension is concentrated by centrifugation or filtration to the desired volume, and lyophilised. The lyophilisate, resuspended in appropriate solution with appropriate pH, contains nano- or micro particles.

Figure 16 shows the release profile of the particles of example 22. The particles of example 22 were administered orally to four rats at a dose of 10 mg/kg body weight. Figure 17 shows the *in-vivo* pharmacokinetics of obtained for the particles of example 22.

### Example 23

1.5g of the polymer Eudragit S100 is dissolved in 10 ml of a mixture of ethyl acetate and DMSO 60/40 (v/v) and the solution is transferred to a double-walled glass vessel (inside height 16.0 cm, inside diameter 3.4 cm). 1.5 ml of DMSO solution containing 350 mg rosuvastatin Ca salt is dispersed by means of a mechanical agitator (Dispermat FT, VMA-Getzmann GmbH, Germany, equipped with a 2.5 cm dissolver disc) in the polymer solution for 15min at 3000 rpm at room temperature. Additionally, 500 mg of the polymer ethyl cellulose in 10 ml of a mixture of ethyl acetate and DMSO 60/40 (v/v) is added and then dispersed by means of a mechanical agitator (Dispermat FT, VMA-Getzmann GmbH, Germany, equipped with a 2.5 cm dissolver disc) in the polymer solution for another 10 minutes at 3000rpm.

50 mL PVA solution (1.0 % (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1.0 % (w/v)) in 100 mmol acetate buffer pH 4.0 containing 25 g sucrose is added. The suspension of nano- and micro particles is agitated with a magnetic stirrer. 100 mL PVA solution (1.0% (w/v)) in 100 mmol acetate buffer pH 4.0 containing 25 g sucrose is added after 15 min, and 250 mL 20 mmol acetate buffer pH 4.0 is added after 30 min, respectively. After 40 min the nano- and micro particles are separated by filtration. Particles are re-constituted in a 1 L two-necked flask by addition of 100 ml PVA solution (1.0% (w/v)) in 100 mmol acetate buffer pH 4.0 containing 25.0 g sucrose. The solvent ethyl acetate is eliminated at room temperature by applying a vacuum and glycofurol is removed by extraction. After 2 h 50 mL PVA solution (1.0% (w/v)) in 100 mmol acetate buffer pH 4.0 containing 12.5 g sucrose is added and vacuum is applied for another 60 min. The nano- and micro particles are washed with 2 L 20 mmol acetate buffer pH 4.0 containing 0.5% PVA centrifugation or filtration. Finally the suspension is concentrated by centrifugation or filtration to the desired volume, and lyophilised. The lyophilisate, resuspended in appropriate solution with appropriate pH, contains nano- or micro particles.

### Example 24

2.5 g of the polymer Eudragit S100 is dissolved in 15 ml of a mixture of ethyl acetate and dimethylsulfoxide (DMSO) 70/30 (v/v) and the solution is transferred to a double-walled glass vessel (inside height 16.0 cm, inside diameter 3.4 cm). 2.0 ml of NMP solution containing 500 mg quetiapine fumarate is dispersed by means of a mechanical agitator (Dispermat FT, VMA-Getzmann GmbH, Germany, equipped with a 2.5 cm dissolver disc) in the polymer solution for 13min at 2000 rpm and for 20 min at 3000 rpm at room temperature.

50 mL PVA solution (1.0% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1.0% (w/v)) in 100 mmol acetate buffer pH 4.0 containing 25 g sucrose is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1.0% (w/v)) in 100 mmol acetate buffer pH 4.0 containing 25.0 g sucrose. The solvent ethyl acetate is eliminated at room temperature by applying a vacuum and DMSO and NMP are removed by extraction. After 2 h the nano- and micro particles are washed with 2 L 20 mmol acetate buffer pH 4.0 by centrifugation or filtration. Finally the suspension is concentrated by centrifugation or filtration to the desired volume, and lyophilised. The lyophilisate, resuspended in appropriate solution with appropriate pH, contains nano- or micro particles.

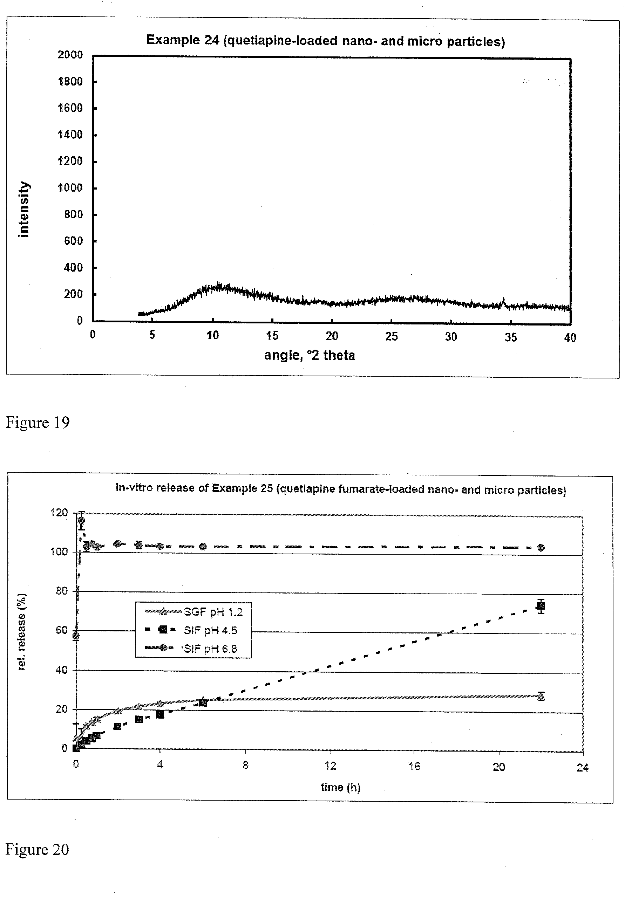

Powder X-ray diffraction is used to determine the state of the active agent in the prepared formulation. Figure 19 exhibits the X-ray diffractogramm of the particles of Example 24. The X-ray diffractogramm of quetiapine fumarate as reference is shown in Figure 18.

### Example 25

2.0 g of the polymer HPMCP HP 55S is dissolved in 15 ml of a mixture of ethyl acetate and DMSO 60/40 (v/v) and the solution is transferred to a double-walled glass vessel (inside height 16.0 cm, inside diameter 3.4 cm). 2.0 ml of DMSO solution containing 350 mg quetiapine fumarate is dispersed by means of a mechanical agitator (Dispermat FT, VMA-Getzmann GmbH, Germany, equipped with a 2.5 cm dissolver disc) in the polymer solution for 20 min at 3000 rpm at room temperature.

50 mL PVA solution (1.0 % (w/v)) in 100 mmol succinate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1.0 % (w/v)) in 100 mmol succinate buffer pH 4.0 containing 25 g sucrose is added. The suspension of nano- and micro particles is agitated with a magnetic stirrer. 100 mL PVA solution (0.5% (w/v)) in 100 mmol succinate buffer pH 4.0 containing 25 g sucrose is added after 15 min, and 250 mL succinate buffer pH 4.0 is added after 30 min, respectively. After 40 min the nano- and micro particles are separated by filtration. Particles are re-constituted in a 1 L two-necked flask by addition of 100 ml PVA solution (0.5 % (w/v)) in 100 mmol succinate buffer pH 4.0 containing 25.0 g sucrose. The solvent ethyl acetate is eliminated at room temperature by applying a vacuum and DMSO is removed by extraction. After 2 h 50 mL PVA solution (0.5% (w/v)) in 100 mmol succinate buffer pH 4.0 containing 12.5 g sucrose is added and vacuum is applied for another 60 min. The nano- and micro particles are washed with 2 L 20 mmol succinate buffer pH 4.0 containing 0.5% PVA centrifugation or filtration. Finally the suspension is concentrated by centrifugation or filtration to the desired volume, and lyophilised. The lyophilisate, resuspended in appropriate solution with appropriate pH, contains nano- or micro particles.

The *in-vitro* release profile of the particles of Example 25 is shown in Figure 20.

### Example 26

0.5 g of the polymer Eudragit S100 is dissolved in 5 ml of a mixture of ethyl acetate and dimethylsulfoxide (DMSO) 60/40 (v/v) and 1.5 g HPMC HP 55S is solved in 10 ml of the of a mixture of ethyl acetate and dimethylsulfoxide (DMSO) 60/40 (v/v) . The both polymer solutions are transferred to a double-walled glass vessel (inside height 16.0 cm, inside diameter 3.4 cm). 2.0 ml of DMSO solution containing 350 mg quetiapine fumarate is dispersed by means of a mechanical agitator (Dispermat FT, VMA-Getzmann GmbH, Germany, equipped with a 2.5 cm dissolver disc) in the polymer solution for 15 min at 3000 rpm at room temperature.

50 mL PVA solution (1.0% (w/v)) in 100 mmol succinate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1.0% (w/v)) in 100 mmol succinate buffer pH 4.0 containing 25 g sucrose is added. The suspension of nano- and micro particles is agitated with a magnetic stirrer. 100 mL PVA solution (0.5% (w/v)) in 100 mmol succinate buffer pH 4.0 containing 25 g sucrose is added after 15 min, and 250 mL water pH 4.0 is added after 30 min, respectively. After 40 min the nano- and micro particles are separated by filtration. Particles are re-constituted in a 1 L two-necked flask by addition of 100 ml PVA solution (0.5% (w/v)) in 100 mmol succinate buffer pH 4.0 containing 25.0 g sucrose. The solvent ethyl acetate is eliminated at room temperature by applying a vacuum and DMSO is removed by extraction. After 2 h 50 mL PVA solution (0.5% (w/v)) in 100 mmol succinate buffer pH 4.0 containing 12.5 g sucrose is added and vacuum is applied for another 60 min. The nano- and micro particles are washed with 2 L 20 mmol succinate buffer pH 4.0 containing 0.5% PVA by centrifugation or filtration. Finally the suspension is concentrated by centrifugation or filtration to the desired volume, and lyophilised. The lyophilisate, resuspended in appropriate solution with appropriate pH, contains nano- or micro particles.

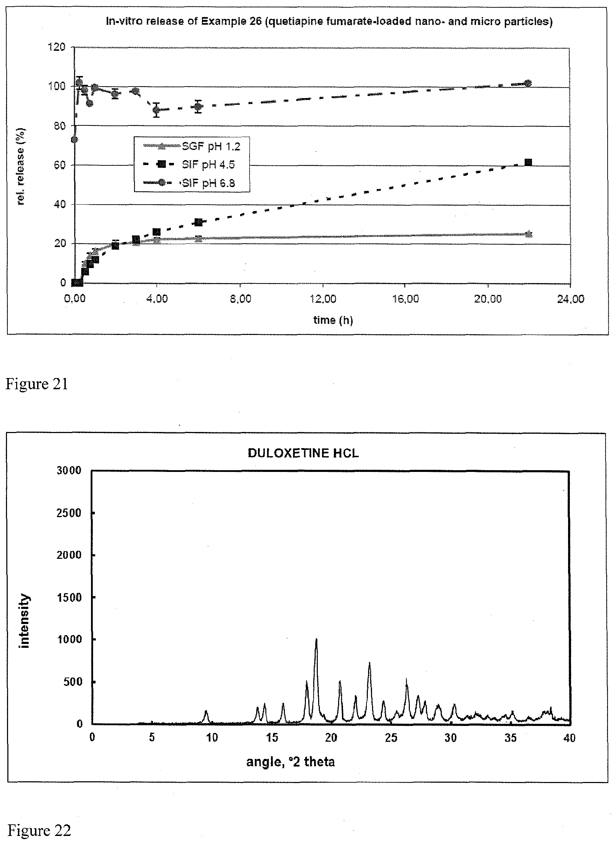

The in-vitro release profile of the particles of Example 26 is shown in Figure 21.

### Example 27

0.5 g of the Eudragit polymer FS30D is dissolved in 5 ml of a mixture of ethyl acetate and dimethylsulfoxide (DMSO) 60/40 (v/v). 1.5 g polymer HPMCP HP55S is dissolved in 10 ml of a mixture ethylacetate and dimethylsulfoxid (DMSO) 60/40 (v/v) and the both solution is transferred to a double-walled glass vessel (inside height 16.0 cm, inside diameter 3.4 cm). 2.0 ml of DMSO solution containing 350 mg quetiapine fumarate is dispersed by means of a mechanical agitator (Dispermat FT, VMA-Getzmann GmbH, Germany, equipped with a 2.5 cm dissolver disc) in the polymer solution for 20 min at 3000 rpm at room temperature.

50 mL PVA solution (0.5% (w/v)) in 100 mmol succinate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1.0% (w/v)) in 100 mmol succinate buffer pH 4.0 containing 25 g sucrose is added. The suspension of nano- and micro particles is agitated with a magnetic stirrer. 100 mL PVA solution (0.05% (w/v)) in 100 mmol succinate buffer pH 4.0 containing 25 g sucrose is added after 15 min, and 250 mL water pH 3.6 is added after 30 min, respectively. After 40 min the nano- and micro particles are separated by filtration. Particles are re-constituted in a 1 L two-necked flask by addition of 100 ml PVA solution (0.5% (w/v)) in 100 mmol succinate buffer pH 4.0 containing 25.0 g sucrose. The solvent ethyl acetate is removed at room temperature by applying a vacuum and DMSO is removed by extraction. After 2 h 50 mL PVA solution (0.5% (w/v)) in 100 mmol succinate buffer pH 4.0 containing 12.5 g sucrose is added and vacuum is applied for another 60 min. The nano- and micro particles are washed with 2 L 20 mmol succinate buffer pH 4.0 by centrifugation or filtration. Finally the suspension is concentrated by centrifugation or filtration to the desired volume, and lyophilised. The lyophilisate, resuspended in appropriate solution with appropriate pH, contains nano- or micro particles.

### Example 28

2.0 g of the polymer CAP is dissolved in 15 ml of a mixture of ethyl acetate and dimethylsulfoxide (DMSO) 60/40 (v/v) and the solution is transferred to a double-walled glass vessel (inside height 16.0 cm, inside diameter 3.4 cm). 2.0 ml of DMSO solution containing 350 mg quetiapine fumarate is dispersed by means of a mechanical agitator (Dispermat FT, VMA-Getzmann GmbH, Germany, equipped with a 2.5 cm dissolver disc) in the polymer solution for 20 min at 3000 rpm at room temperature.

50 mL PVA solution (0.5% w/v)) in 100 mmol succinate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (0.5% (w/v)) in 100 mmol succinate buffer pH 4.0 containing 25 g sucrose is added. The suspension of nano- and micro particles is agitated with a magnetic stirrer. 100 mL PVA solution (0.05% (w/v)) in 100 mmol succinate buffer pH 4.0 containing 25 g sucrose is added after 15 min, and 250 mL 20 mmol acetate buffer pH 3.6 is added after 30 min, respectively. After 40 min the nano- and micro particles are separated by filtration. Particles are re-constituted in a 1 L two-necked flask by addition of 100 ml PVA solution (0.5% (w/v)) in 100 mmol succinate buffer pH 4.0 containing 25.0 g sucrose. The solvent ethyl acetate is eliminated at room temperature by applying a vacuum and DMSO is removed by extraction. After 2 h 50 mL PVA solution (0.5% (w/v)) in 100 mmol succinate buffer pH 4.0 containing 12.5 g sucrose is added and vacuum is applied for another 60 min. The nano-and micro particles are washed with 2 L 20 mmol acetate buffer pH 4.0 by centrifugation or filtration. Finally the suspension is concentrated by centrifugation or filtration to the desired volume, and lyophilised. The lyophilisate, resuspended in appropriate solution with appropriate pH, contains nano- or micro particles.

### Example 29

2.0 g of the polymer HPMCP HP 55S is dissolved in 15 ml of a mixture of ethyl acetate and dimethylsulfoxide (DMSO) 70/30 (v/v) and the solution is transferred to a double-walled glass vessel (inside height 16.0 cm, inside diameter 3.4 cm). 3.0 ml of DMSO solution containing 350 mg duloxetine hydrochloride is dispersed by means of a mechanical agitator (Dispermat FT, VMA-Getzmann GmbH, Germany, equipped with a 2.5 cm dissolver disc) in the polymer solution for 22 min at 3000 rpm at room temperature.

50 mL Poloxamer 188 solution (3.0% (w/v)) in 100 mmol acetate buffer pH 4.0 containing 2.5 g NaCl is then added as continuous phase during agitation at 3000 rpm. After about 60 seconds of agitation, the suspension of nano- and micro particles is transferred to a beaker and 100 mL Poloxamer solution (16% (w/v)) in 100 mmol acetate buffer pH 4.0 containing 5 g NaCl is added. The suspension of nano- and micro particles is agitated with a magnetic stirrer. 300 mL 20 mmol acetate buffer pH 3.6 is added after 10 min. After 20 min the nano- and micro particles are separated by filtration. Particles are re-constituted in a 1 L two-necked flask by addition of 100 ml Poloxamer solution (16% (w/v)) in 100 mmol acetate buffer pH 4.0 containing 5.0 g NaCl. The solvent ethyl acetate is eliminated at room temperature by applying a vacuum and DMSO is removed by extraction. After 2 h the nano- and micro particles are washed with 2 L 20 mmol acetate buffer pH 4.0 containing 0.1% Poloxamer 188 by centrifugation or filtration. Finally the suspension is concentrated by centrifugation or filtration to the desired volume, and lyophilised. The lyophilisate, resuspended in appropriate solution with appropriate pH, contains nano- or micro particles.

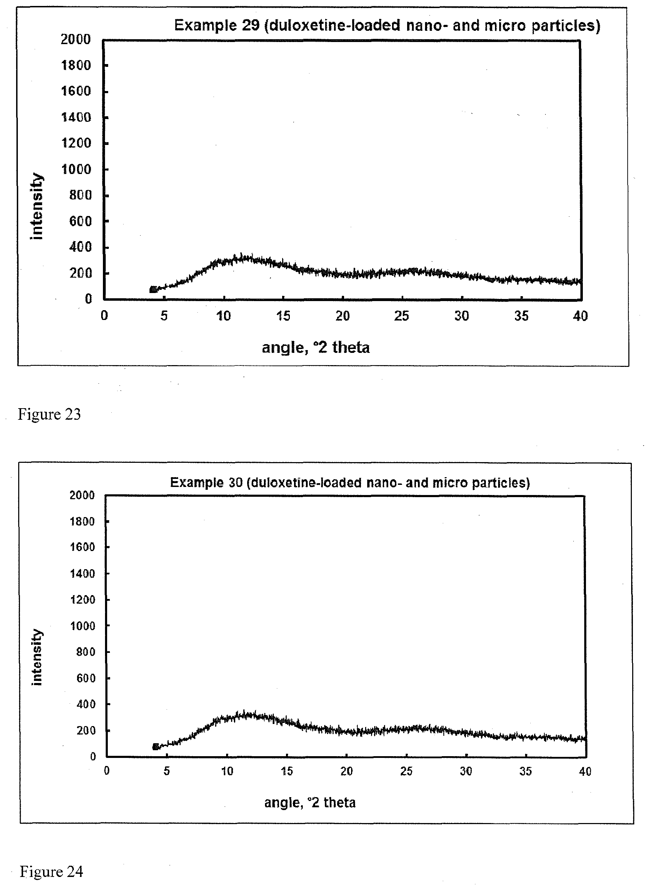

Powder X-ray diffraction is used to determine the state of the active agent in the prepared formulation (Figure 23), as a reference the X-ray diffractogramm of duloxetine hydrochloride is shown in Figure 22.

### Example 30

2.0 g of the polymer Eudragit L100-55 is dissolved in 15 ml of a mixture of ethyl acetate and DMSO 70/30 (v/v) and the solution is transferred to a double-walled glass vessel (inside height 16.0 cm, inside diameter 3.4 cm). 1.2 ml of NMP solution containing 700 mg duloxetine hydrochloride is dispersed by means of a mechanical agitator (Dispermat FT, VMA-Getzmann GmbH, Germany, equipped with a 2.5 cm dissolver disc) in the polymer solution for 25 min at 3000 rpm at room temperature.

50 mL PVA solution (0.5% (w/v)) in 100 mmol acetate buffer pH 4.0 containing 2.5 g NaCl is then added as continuous phase during agitation at 3000 rpm. After about 60 seconds of agitation, the suspension of nano- and micro particles is transferred to a beaker and 100 mL PVA solution (0.5% (w/v)) in 100 mmol acetate buffer pH 4.0 containing 5 g NaCl is added.

The suspension of nano- and micro particles is agitated with a magnetic stirrer. After 30 min the nano- and micro particles are separated by centrifugation or filtration. Particles are re-constituted in a 1L two-necked flask by addition of 100 ml PVA solution (0.5% (w/v)) in 100 mmol acetate buffer pH 4.0 containing 5.0 g NaCl. The solvent ethyl acetate is eliminated at room temperature by applying a vacuum and DMSO is removed by extraction. After 2 h nano- and micro particles are washed with 2 L 10 mmol acetate buffer pH 3.6 and the suspension is concentrated by centrifugation or filtration to the desired volume. Finally the nano- and micro particles are lyophilised. The lyophilisate, resuspended in appropriate solution with appropriate pH, contains nano- or micro particles.

The X-ray diffractogramm of the particles of example 30 is shown in Figure 24.

### Example 31

2.0 g of the polymer Eudragit S100 is dissolved in 15 ml of a mixture of ethyl acetate and DMSO 70/30 (v/v) and the solution is transferred to a double-walled glass vessel (inside height 16.0 cm, inside diameter 3.4 cm). 1.0 ml of NMP solution containing 350 mg duloxetine hydrochloride is dispersed by means of a mechanical agitator (Dispermat FT, VMA-Getzmann GmbH, Germany, equipped with a 2.5 cm dissolver disc) in the polymer solution for 20 min at 3000 rpm at room temperature.

50 mL PVA solution (2.0% (w/v)) in 100 mmol acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (2.0% (w/v)) in 100 mmol acetate buffer pH 4.0 containing 25 g sucrose is added. The suspension of nano- and micro particles is agitated with a magnetic stirrer. After 30 min the nano- and micro particles are separated by centrifugation or filtration. Particles are re-constituted in a 1 L two-necked flask by addition of 100 ml PVA solution (2.0% (w/v)) in 100 mmol acetate buffer pH 4.0 containing 25 g sucrose. The solvent ethyl acetate is eliminated at room temperature by applying a vacuum and DMSO is removed by extraction. After 2 h nano- and micro particles are washed with 2 L 10 mmol acetate buffer pH 3.6 containing 0.25% Poloxamer 188 by filtration or centrifugation. Finally the suspension is concentrated by centrifugation or filtration to the desired volume, and lyophilized. The lyophilisate, resuspended in appropriate solution with appropriate pH, contains nano- or micro particles.

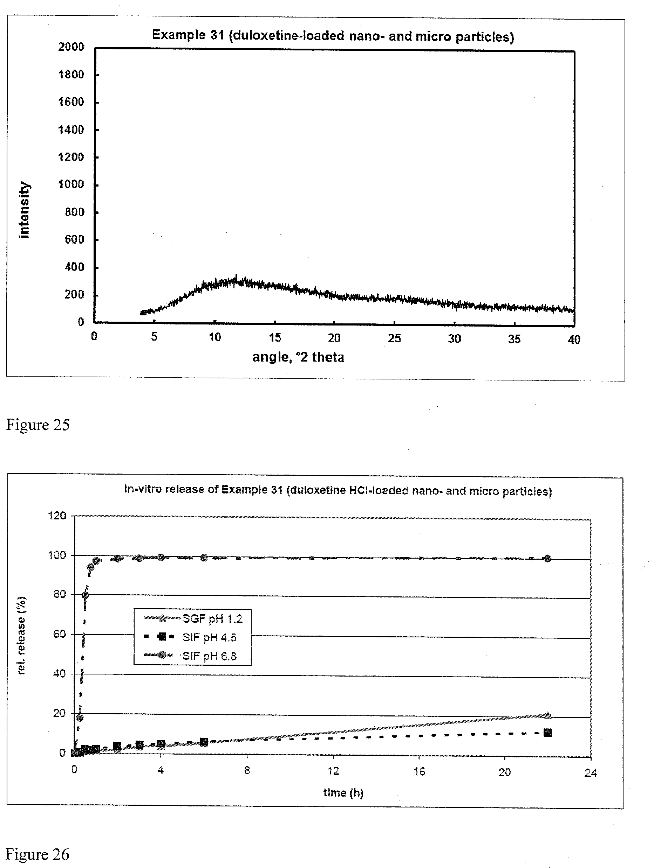

The in-vitro release profile of the partilcles of example 31 is shown in Figure 26. The X-ray diffractogramm of the particles of example 31 is shown in Figure 25.

### Example 32

2.0 g of the polymer Eudragit S100 is dissolved in 15 ml of a mixture of ethyl acetate and glycofurol 60/40 (v/v) and the solution is transferred to a double-walled glass vessel (inside height 16.0 cm, inside diameter 3.4 cm). 1.0 ml of glycofurol solution containing 350 mg duloxetine hydrochloride is dispersed by means of a mechanical agitator (Dispermat FT, VMA-Getzmann GmbH, Germany, equipped with a 2.5 cm dissolver disc) in the polymer solution for 15 min at 3000 rpm at room temperature.

50 mL PVA solution (2.0% (w/v)) in 100 mmol succinate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (2.0% (w/v)) in 100 mmol sucinate buffer pH 4.0 containing 25 g sucrose is added. The suspension of nano- and micro particles is agitated with a magnetic stirrer. After 30 min the nano- and micro particles are separated by centrifugation or filtration. Particles are re-constituted in a 1 L two-necked flask by addition of 100 ml PVA solution (2.0% (w/v)) in 100 mmol succinate buffer pH 4.0 containing 25 g sucrose. The solvent ethyl acetate is eliminated at room temperature by applying a vacuum and glycofurol is removed by extraction. After 2 h nano- and micro particles are washed with 2 L 10 mmol acetate buffer pH 3.6 containing 0.25% Poloxamer 188 by filtration or centrifugation. The suspension is concentrated by centrifugation or filtration to the desired volume, and lyophilized. The lyophilisate, resuspended in appropriate solution with appropriate pH, contains nano- or micro particles.

### Example 33

2.0 g of the polymer Eudragit S100 is dissolved in 15 ml of a mixture of ethyl acetate and DMSO 60/40 (v/v) and the solution is transferred to a double-walled glass vessel (inside height 16.0 cm, inside diameter 3.4 cm). 1.0 ml of DMSO solution containing 350 mg Asenapine maleate is dispersed by means of a mechanical agitator (Dispermat FT, VMA-Getzmann GmbH, Germany, equipped with a 2.5 cm dissolver disc) in the polymer solution for 20 min at 3000 rpm at room temperature.

80 mL PVA solution (1.0% (w/v)) in 100 mmol Acetate buffer pH 4.0 containing 20.0 g sucrose is then added as continuous phase during agitation at 3000 rpm. After about 60 seconds of agitation, the suspension of nano- and micro particles is transferred to a beaker and 100 mL PVA solution (1.0% (w/v)) in 100 mmol acetate buffer pH 4.0 containing 25 g sucrose is added. The suspension of nano- and micro particles is agitated with a magnetic stirrer. After 30 min the nano- and micro particles are separated by centrifugation or filtration. Particles are re-constituted in a 1 L two-necked flask by addition of 100 ml PVA solution (1.0% (w/v)) in 100 mmol Acetate buffer pH 4.0 containing 25 g sucrose. The solvent ethyl acetate is eliminated at room temperature by applying a vacuum and DMSO is removed by extraction. After 2 h nano- and micro particles are washed with 2 L 10 mmol acetate buffer pH 3.8 containing 0.25% Poloxamer 188 by filtration or centrifugation. The suspension is concentrated by centrifugation or filtration to the desired volume, and lyophilized. The lyophilisate, resuspended in appropriate solution with appropriate pH, contains nano- or micro particles.

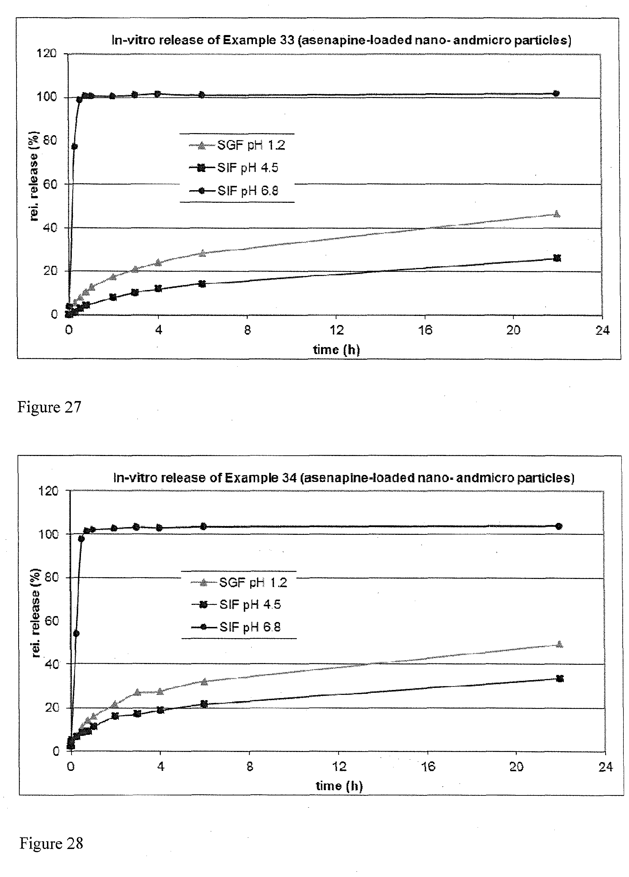

The in-vitro relase profile of the particles of example 33 is shown in Fig. 27.

### Example 34

2.0 g of the polymer Eudragit S100 is dissolved in 15 ml of a mixture of ethyl acetate and DMSO 60/40 (v/v) and the solution is transferred to a double-walled glass vessel (inside height 16.0 cm, inside diameter 3.4 cm). 1.0 ml of DMSO solution containing 350 mg Asenapine maleate is dispersed by means of a mechanical agitator (Dispermat FT, VMA-Getzmann GmbH, Germany, equipped with a 2.5 cm dissolver disc) in the polymer solution for 20 min at 3000 rpm at room temperature.

80 mL PVA solution (1.0% (w/v)) in 100 mmol Acetate buffer pH 4.0 containing 20.0 g sucrose is then added as continuous phase during agitation at 3000 rpm. After about 60 seconds of agitation, the suspension of nano- and micro particles is transferred to a beaker and 100 mL PVA solution (1.0% (w/v)) in 100 mmol acetate buffer pH 4.0 containing 25 g sucrose is added. The suspension of nano- and micro particles is agitated with a magnetic stirrer. After 30 min the nano- and micro particles are separated by centrifugation or filtration. Particles are re-constituted in a 1 L two-necked flask by addition of 100 ml PVA solution (1.0% (w/v)) in 100 mmol Acetate buffer pH 4.0 containing 25 g sucrose. The solvent ethyl acetate is eliminated at room temperature by applying a vacuum and DMSO is removed by extraction. After 2 h nano- and micro particles are washed with 2 L water pH 3.8 by filtration or centrifugation. Finally the nano- and micro particles are coated with 400 ml 1% chitosan solution pH 4.0, for 2 hours, filtered, washed and lyophilized. The lyophilisate, resuspended in appropriate solution with appropriate pH, contains nano- or micro particles.

The in-vitro release profile of example the particles of example 34 is shown in Figure 28.

### Example 35

2.0 g of the polymer Eudragit S100 is dissolved in 15 ml of a mixture of ethyl acetate and NMP 60/40 (v/v) and the solution is transferred to a double-walled glass vessel (inside height 16.0 cm, inside diameter 3.4 cm). 1.0 ml of NMP solution containing 350 mg Aripiprazole is dispersed by means of a mechanical agitator (Dispermat FT, VMA-Getzmann GmbH, Germany, equipped with a 2.5 cm dissolver disc) in the polymer solution for 20 min at 3000 rpm at room temperature.

50 mL PVA solution (1.0% (w/v)) in 100 mmol Acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1.0% (w/v)) in 100 mmol acetate buffer pH 4.0 containing 25 g sucrose is added. The suspension of nano- and micro particles is agitated with a magnetic stirrer. After 30 min the nano- and micro particles are separated by centrifugation or filtration. Particles are re-constituted in a 1 L two-necked flask by addition of 100 ml PVA solution (1.0% (w/v)) in 100 mmol Acetate buffer pH 4.0. The solvent ethyl acetate is eliminated at room temperature by applying a vacuum and NMP is removed by extraction. After 2 hours nano- and micro particles are washed with 2 L water pH 3.8 by filtration or centrifugation. Finally the nano- and micro particles are coated with 750 ml 1% chitosan solution pH 4.0 in acetate buffer pH 4.0 for 2 hours, filtered, washed and lyophilized. The lyophilisate, resuspended in appropriate solution with appropriate pH, contains nano- or micro particles.

### Example 36

2.0 g of the polymer Eudragit L100-55 is dissolved in 15 ml of a mixture of ethyl acetate and NMP 60/40 (v/v) and the solution is transferred to a double-walled glass vessel (inside height 16.0 cm, inside diameter 3.4 cm). 1.0 ml of NMP solution containing 350 mg Aripiprazole is dispersed by means of a mechanical agitator (Dispermat FT, VMA-Getzmann GmbH, Germany, equipped with a 2.5 cm dissolver disc) in the polymer solution for 20 min at 3000 rpm at room temperature.

50 mL PVA solution (1.0% (w/v)) in 100 mmol Acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1.0% (w/v)) in 100 mmol acetate buffer pH 4.0 containing 25 g sucrose is added. The suspension of nano- and micro particles is agitated with a magnetic stirrer. After 30 min the nano- and micro particles are separated by centrifugation or filtration. Particles are re-constituted in a 1 L two-necked flask by addition of 100 ml PVA solution (1.0% (w/v)) in 100 mmol Acetate buffer pH 4.0. The solvent ethyl acetate is eliminated at room temperature by applying a vacuum and NMP is removed by extraction. After 2 hours nano- and micro particles are washed with 2 L water pH 3.8 by filtration or centrifugation. Finally the nano- and micro particles are coated with 750 ml 1% chitosan solution pH 4.0 in acetate buffer pH 4.0 for 2 hours, filtered, washed and lyophilized. The lyophilisate, resuspended in appropriate solution with appropriate pH, contains nano- or micro particles.

### Example 37

2.0 g of the polymer Eudragit S100 is dissolved in 15 ml of a mixture of ethyl acetate and DMSO 60/40 (v/v) and the solution is transferred to a double-walled glass vessel (inside height 16.0 cm, inside diameter 3.4 cm). 1.5 ml of DMSO solution containing 500 mg Raloxifene hydrochloride is dispersed by means of a mechanical agitator (Dispermat FT, VMA-Getzmann GmbH, Germany, equipped with a 2.5 cm dissolver disc) in the polymer solution for 20 min at 3000 rpm at room temperature.

50 mL PVA solution (1.0% (w/v)) in 100 mmol Acetate buffer pH 4.0 containing 12.5 g sucrose is then added as continuous phase during agitation at 3000 rpm. After about 60 seconds of agitation, the suspension of nano- and micro particles is transferred to a beaker and 100 mL PVA solution (1.0% (w/v)) in 100 mmol acetate buffer pH 4.0 containing 25 g sucrose is added. The suspension of nano- and micro particles is agitated with a magnetic stirrer. After 30 min the nano- and micro particles are separated by centrifugation or filtration. Particles are re-constituted in a 1 L two-necked flask by addition of 200 ml PVA solution (1.0 % (w/v)) in 100 mmol acetate buffer pH 4.0. The solvents are eliminated at room temperature by applying extraction. After 2 h nano- and micro particles are washed with 2 L water pH 3.8 by filtration. Finally the nano- and micro particles are coated with 750 ml 1% chitosan solution pH 4.0 in acetate buffer pH 4.0 for 2 hours, filtered, washed and lyophilized The lyophilisate, resuspended in appropriate solution with appropriate pH, contains nano- or micro particles.

### Example 38

1.0 g of the polymer Ethylcellulose S100 is dissolved in 15 ml of a mixture of ethyl acetate and dimethylsulfoxide (DMSO) 60/40 (v/v) and the solution is transferred to a double-walled glass vessel (inside height 16.0 cm, inside diameter 3.4 cm). 1.0 ml DMSO solution containing 350 mg raloxifene hydrochloride and 100 mg of Polymer HPMC AS-HG is dispersed by means of a mechanical agitator (Dispermat FT, VMA-Getzmann GmbH, Germany, equipped with a 2.5 cm dissolver disc) in the polymer solution for 15 minutes at 3000 rpm at room temperature.

80 mL PVA solution (1% (w/v)) in 100 mmol phosphate buffer pH 6.0 containing 20.0 g sucrose is added as continuous phase during agitation at 3000 rpm. After about 60 seconds of agitation, the suspension of nano- and micro particles is transferred to a beaker and 100 mL PVA solution (1% (w/v)) in 100 mmol phosphate buffer pH 6.0 containing 25.0 g sucrose is added. The suspension of nano- and micro particles is agitated with a magnetic stirrer. After 60 min the nano- and micro particles are separated by centrifugation. Particles are re-constituted in a 1 L two-necked flask by addition of 80 ml PVA solution (1% (w/v)) in 100 mmol phosphate buffer pH 6.0 containing 25.0 g sucrose. The solvent ethyl acetate is eliminated by applying a vacuum and DMSO is removed by extraction. After 45 min 50 mL PVA solution (1% (w/v)) in 100 mmol phosphate buffer pH 6.0 containing 12.5 g sucrose is added and evaporation is continued for 150 min. Nano- and micro particles are washed washed with 1 L phosphate buffer pH 6.0 And concentrated by centrifugation or filtration to the desired volume. Finally the nano- and micro particles are coated with 500 ml 10 mmol acetate buffer pH 4.0 containing 5g chitosan for 2 hours, filtered, washed and lyophilized. The lyophilisate, resuspended in appropriate solution with appropriate pH, contains nano or micro particles.

### Example 39

2.0 g of the polymer Eudragit S100 is dissolved in 15 ml of a mixture of ethyl acetate and dimethylsulfoxide (DMSO) 70/30 (v/v) and the solution is transferred to a double-walled glass vessel (inside height 16.0 cm, inside diameter 3.4 cm). 1.0 ml of NMP solution containing 350 mg quetiapine fumarate and 100 mg of polymer HPMC AS-HG is dispersed by means of a mechanical agitator (Dispermat FT, VMA-Getzmann GmbH, and Germany, equipped with a 2.5 cm dissolver disc) in the polymer solution for 13 min at 2000 rpm and for 15 min at 3000 rpm at room temperature.

50 mL PVA solution (1.0% (w/v)) in 100 mmol acetate buffer pH 4.0 containing 2.5 g NaCl is then added as continuous phase during agitation at 3000 rpm. After about 60 seconds of agitation, the suspension of nano- and micro particles is transferred to a beaker and 100 mL PVA solution (1.0% (w/v)) in 100 mmol acetate buffer pH 4.0 containing 5.0 g NaCl 25 g sucrose is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1.0% (w/v)) in 100 mmol acetate buffer pH 4.0 containing 5.0 g NaCl. The solvent ethyl acetate is eliminated at room temperature by applying a vacuum and DMSO and NMP are removed by extraction. After 2 hours the nano- and micro particles are washed with 2 L 20 mmol acetate buffer pH 4.0 by centrifugation or filtration. Finally the suspension is concentrated by centrifugation or filtration to the desired volume, and lyophilised. The lyophilisate, resuspended in appropriate solution with appropriate pH, contains nano- or micro particles.

### Example 40

2.0 g of the polymer Ethylcellulose is dissolved in 15 ml of a mixture of ethyl acetate and dimethylsulfoxide (DMSO) and the solution is transferred to a double-walled glass vessel (inside height 16.0 cm, inside diameter 3.4 cm). 1.0 ml of NMP solution containing 350 mg quetiapine fumarate and 100 mg of polymer HPMC AS-HG is dispersed by means of a mechanical agitator (Dispermat FT, VMA-Getzmann GmbH, and Germany, equipped with a 2.5 cm dissolver disc) in the polymer solution for 13 min at 2000 rpm and for 15 min at 3000 rpm at room temperature.

80 mL PVA solution (1.0% (w/v)) in 100 mmol acetate buffer pH 4.0 containing 20.0 g Sucrose is then added as continuous phase during agitation at 3000 rpm. After about 60 seconds of agitation, the suspension of nano- and micro particles is transferred to a beaker and 100 mL PVA solution (1.0% (w/v)) in 100 mmol acetate buffer pH 4.0 containing 25 g sucrose is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1.0% (w/v)) in 100 mmol acetate buffer pH 4.0 containing 25.0 g sucrose. The solvent ethyl acetate is eliminated at room temperature by applying a vacuum and DMSO and NMP are removed by extraction. After 2 h the nano- and micro particles are washed with 2 L 20 mmol acetate buffer pH 4.0 by centrifugation or filtration. Finally the suspension is concentrated by centrifugation or filtration to the desired volume, and lyophilised. The lyophilisate, resuspended in appropriate solution with appropriate pH, contains nano- or micro particles.

### Example 41

2.0 g of the polymer Ethylcellulose is dissolved in 15 ml of a mixture of ethyl acetate and dimethylsulfoxide (DMSO) and the solution is transferred to a double-walled glass vessel (inside height 16.0 cm, inside diameter 3.4 cm). 1.0 ml of 2 ml dimethylsulfoxide (DMSO) solution containing 350 mg quetiapine fumarate and 100 mg of polymer HPMC AS-HG is dispersed by means of a mechanical agitator (Dispermat FT, VMA-Getzmann GmbH, and Germany, equipped with a 2.5 cm dissolver disc) in the polymer solution for 13 min at 2000 rpm and for 15 min at 3000 rpm at room temperature.

80 mL PVA solution (1.0% (w/v)) in 100 mmol acetate buffer pH 4.0 containing 20.0 g sucrose is then added as continuous phase during agitation at 3000 rpm. After about 60 seconds of agitation, the suspension of nano- and micro particles is transferred to a beaker and 100 mL PVA solution (1.0% (w/v)) in 100 mmol acetate buffer pH 4.0 containing 25 g sucrose is added. The suspension of nano- and micro particles is agitated with a magnetic stirrer. After 30 min the nano- and micro particles are separated by centrifugation. Particles are re-constituted in a 1 L two-necked flask by addition of 100 ml PVA solution (1.0% (w/v)) in 100 mmol acetate buffer pH 4.0 containing 25.0 g sucrose. The solvent ethyl acetate is eliminated at room temperature by applying a vacuum and DMSO and NMP are removed by extraction. After 2 h the nano- and micro particles are washed with 2 L 20 mmol acetate buffer pH 4.0 by centrifugation or filtration. Finally the suspension is concentrated by centrifugation or filtration to the desired volume, and lyophilised. The lyophilisate, resuspended in appropriate solution with appropriate pH, contains nano- or micro particles.

### Example 42

1500 mg of the polymer Eudragit S100 is dissolved in 10 ml of a mixture of ethyl acetate and dimethylsulfoxide (DMSO) 60/40 % (v/v) and the solution is transferred to a double-walled glass vessel (inside height 16.0 cm, inside diameter 3.4 cm). 1.0 ml DMSO solution containing 350 mg clonidine hydrochloride is dispersed by means of a mechanical agitator (Dispermat FT, VMA-Getzmann GmbH, Germany, equipped with a 2.5 cm dissolver disc) in the polymer solution for 10 minutes at 3000 rpm at room temperature. Additionally, 500 mg of the polymer ethyl cellulose in 10 ml of a mixture of ethyl acetate and DMSO 60/40 % (v/v) is added and then dispersed by means of a mechanical agitator (Dispermat FT, VMA-Getzmann GmbH, Germany, equipped with a 2.5 cm dissolver disc) in the polymer solution for another 10 minutes at 3000rpm.

80 mL PVA solution (1% (w/v)) in 100 mmol acetate buffer pH 4.0 containing 20.0g sucrose is added as continuous phase during agitation at 3000 rpm. After about 60 seconds of agitation, the suspension of nano- and micro particles is transferred to a beaker and 100 mL PVA solution (1% (w/v)) in 100 mmol acetate buffer pH 4.0 containing 25 g sucrose is added. The suspension of nano- and micro particles is agitated with a magnetic stirrer. After 15 min 100 mL PVA solution (1% (w/v)) in 100 mmol acetate buffer pH 4.0 containing 25 g sucrose is added and after another 15 min the nano- and micro particles are separated by filtration. Particles are re-constituted in a 1 L two-necked flask by addition of 100 ml PVA solution (1% (w/v)) in 100 mmol acetate buffer pH 4.0 containing 25.0 g sucrose. The solvent ethyl acetate is eliminated by applying a vacuum and DMSO is removed by extraction. After 150 min 50 mL PVA solution (1% (w/v)) in 100 mmol acetate buffer pH 4.0 containing 12.5 g sucrose is added and evaporation is continued for 60 min. Nano- and micro particles are washed with 1 L 20 mm acetate buffer pH 4.0 and concentrated by centrifugation or filtration to the desired volume. Finally the concentrated nano- and micro particle suspension is lyophilized. The lyophilisate, resuspended in appropriate solution with appropriate pH, contains nano or micro particles.

### Example 43

1500 mg of the polymer Eudragit S100 is dissolved in 10 ml of a mixture of ethyl acetate and dimethylsulfoxide (DMSO) 60/40 % (v/v) and the solution is transferred to a double-walled glass vessel (inside height 16.0 cm, inside diameter 3.4 cm). 1.0 ml DMSO solution containing 350 mg Carvedilol dispersed by means of a mechanical agitator (Dispermat FT, VMA-Getzmann GmbH, Germany, equipped with a 2.5 cm dissolver disc) in the polymer solution for 10 minutes at 3000 rpm at room temperature. Additionally, 500 mg of the polymer ethyl cellulose in 10 ml of a mixture of ethyl acetate and DMSO 60/40 % (v/v) is added and then dispersed by means of a mechanical agitator (Dispermat FT, VMA-Getzmann GmbH, Germany, equipped with a 2.5 cm dissolver disc) in the polymer solution for another 10 minutes at 3000rpm.

80 mL PVA solution (1% (w/v)) in 100 mmol acetate buffer pH 4.0 containing 20.0 g sucrose is added as continuous phase during agitation at 3000 rpm. After about 60 seconds of agitation, the suspension of nano- and micro particles is transferred to a beaker and 100 mL PVA solution (1% (w/v)) in 100 mmol acetate buffer pH 4.0 containing 25 g sucrose is added. The suspension of nano- and micro particles is agitated with a magnetic stirrer. After 15 min 100 mL PVA solution (1% (w/v)) in 100 mmol acetate buffer pH 4.0 containing 25 g sucrose is added and after another 15 min the nano- and micro particles are separated by filtration. Particles are re-constituted in a 1 L two-necked flask by addition of 100 ml PVA solution (1% (w/v)) in 100 mmol acetate buffer pH 4.0 containing 25.0 g sucrose. The solvent ethyl acetate is eliminated by applying a vacuum and DMSO is removed by extraction. After 150 min 50 mL PVA solution (1% (w/v)) in 100 mmol acetate buffer pH 4.0 containing 12.5 g sucrose is added and evaporation is continued for 60 min. Nano- and micro particles are washed with 1 L 20 mm acetate buffer pH 4.0 and concentrated by centrifugation or filtration to the desired volume. Finally the concentrated nano- and micro particle suspension is lyophilized. The lyophilisate, resuspended in appropriate solution with appropriate pH, contains nano or micro particles.

### Example 44

1500 mg of the polymer Eudragit S100 is dissolved in 10 ml of a mixture of ethyl acetate and dimethylsulfoxide (DMSO) 60/40 % (v/v) and the solution is transferred to a double-walled glass vessel (inside height 16.0 cm, inside diameter 3.4 cm). 1.0 ml DMSO solution containing 350 mg candesartan ciletexil is dispersed by means of a mechanical agitator (Dispermat FT, VMA-Getzmann GmbH, Germany, equipped with a 2.5 cm dissolver disc) in the polymer solution for 10 minutes at 3000 rpm at room temperature. Additionally, 500 mg of the polymer ethyl cellulose in 10 ml of a mixture of ethyl acetate and DMSO 60/40 % (v/v) is added and then dispersed by means of a mechanical agitator (Dispermat FT, VMA-Getzmann GmbH, Germany, equipped with a 2.5 cm dissolver disc) in the polymer solution for another 10 minutes at 3000rpm.

80 mL PVA solution (1% (w/v)) in 100 mmol acetate buffer pH 4.0 containing 20.0 g sucrose is added as continuous phase during agitation at 3000 rpm. After about 60 seconds of agitation, the suspension of nano- and micro particles is transferred to a beaker and 100 mL PVA solution (1% (w/v)) in 100 mmol acetate buffer pH 4.0 containing 25 g sucrose is added. The suspension of nano- and micro particles is agitated with a magnetic stirrer. After 15 min 100 mL PVA solution (1% (w/v)) in 100 mmol acetate buffer pH 4.0 containing 25 g sucrose is added and after another 15 min the nano- and micro particles are separated by filtration. Particles are re-constituted in a 1 L two-necked flask by addition of 100 ml PVA solution (1% (w/v)) in 100 mmol acetate buffer pH 4.0 containing 25.0 g sucrose. The solvent ethyl acetate is eliminated by applying a vacuum and DMSO is removed by extraction. After 150 min 50 mL PVA solution (1% (w/v)) in 100 mmol acetate buffer pH 4.0 containing 12.5 g sucrose is added and evaporation is continued for 60 min. Nano- and micro particles are washed with 1 L 20 mm acetate buffer pH 4.0 and concentrated by centrifugation or filtration to the desired volume. Finally the concentrated nano- and micro particle suspension is lyophilized. The lyophilisate, resuspended in appropriate solution with appropriate pH, contains nano or micro particles.

### Example 45

1500 mg of the polymer Eudragit S100 is dissolved in 10 ml of a mixture of ethyl acetate and dimethylsulfoxide (DMSO) 60/40 % (v/v) and the solution is transferred to a double-walled glass vessel (inside height 16.0 cm, inside diameter 3.4 cm). 1.0 ml DMSO solution containing 350 mg pramipexol is dispersed by means of a mechanical agitator (Dispermat FT, VMA-Getzmann GmbH, Germany, equipped with a 2.5 cm dissolver disc) in the polymer solution for 10 minutes at 3000 rpm at room temperature. Additionally, 500 mg of the polymer ethyl cellulose in 10 ml of a mixture of ethyl acetate and DMSO 60/40 % (v/v) is added and then dispersed by means of a mechanical agitator (Dispermat FT, VMA-Getzmann GmbH, Germany, equipped with a 2.5 cm dissolver disc) in the polymer solution for another 10 minutes at 3000rpm.

80 mL PVA solution (1% (w/v)) in 100 mmol acetate buffer pH 4.0 containing 20.0 g sucrose is added as continuous phase during agitation at 3000 rpm. After about 60 seconds of agitation, the suspension of nano- and micro particles is transferred to a beaker and 100 mL PVA solution (1% (w/v)) in 100 mmol acetate buffer pH 4.0 containing 25 g sucrose is added. The suspension of nano- and micro particles is agitated with a magnetic stirrer. After 15 min 100 mL PVA solution (1% (w/v)) in 100 mmol acetate buffer pH 4.0 containing 25 g sucrose is added and after another 15 min the nano- and micro particles are separated by filtration. Particles are re-constituted in a 1 L two-necked flask by addition of 100 ml PVA solution (1% (w/v)) in 100 mmol acetate buffer pH 4.0 containing 25.0 g sucrose. The solvent ethyl acetate is eliminated by applying a vacuum and DMSO is removed by extraction. After 150 min 50 mL PVA solution (1% (w/v)) in 100 mmol acetate buffer pH 4.0 containing 12.5 g sucrose is added and evaporation is continued for 60 min. Nano- and micro particles are washed with 1 L 20 mm acetate buffer pH 4.0 and concentrated by centrifugation or filtration to the desired volume. Finally the concentrated nano- and micro particle suspension is lyophilized. The lyophilisate, resuspended in appropriate solution with appropriate pH, contains nano or microparticles.

### Brief Description of the Figures:

Figure 1: X-ray diffraction pattern of Raloxifen
Figure 2: X-ray diffraction pattern of the particles of example 1
Figure 3: X-ray diffraction pattern of the particles of example 2
Figure 4: X-ray diffraction pattern of the particles of example 3
Figure 5: In-vitro release profile of the particles of example 1
Figure 6: In-vitro release profile of the particles of example 2
Figure 7: In-vitro release profile of the particles of example 3
Figure 8: In-vitro release profile of the particles of example 4
Figure 9: In-vitro release profile of the particles of example 5
Figure 10: In-vivo release profile of the particles of examples 1, 2, 3, 4 (in rats)
Figure 11: X-ray diffraction pattern of Atorvastatin
Figure 12: X-ray diffraction pattern of the particles of example 6
Figure 13: In-vitro release profile of the particles of example 13
Figure 14: In-vitro release profile of the particles of example 14
Figure 15: In-vitro release profile of the particles of example 21
Figure 16: In-vitro release profile of the particles of example 22
Figure 17: In-vivo release profile of the particles of examples 21, 22 (in rats)
Figure 18: X-ray diffraction pattern of Quetiapine
Figure 19: X-ray diffraction pattern of the particles of example 24
Figure 20: In-vitro release profile of the particles of example 25
Figure 21: In-vitro release profile of the particles of example 26
Figure 22: X-ray diffraction pattern of Dulexetine
Figure 23: X-ray diffraction pattern of the particles of example 29
Figure 24: X-ray diffraction pattern of the particles of example 30
Figure 25: X-ray diffraction pattern of the particles of example 31
Figure 26: In-vitro release profile of the particles of example 31
Figure 27: In-vitro release profile of the particles of example 33
Figure 28: In-vitro release profile of the particles of example 34

## Claims

1. A process for the production of nano- and/or microparticles for use in oral administration,
which particles contain one or more therapeutic agents dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, said process comprising the steps of
a) providing a solution containing
a1) the one or more therapeutic agents in dissolved form;
a2) the one or more polymers in dissolved form, and
a3) a solvent mixture containing
(i) at least one solvent S1 which is fully miscible with water, and which is a solvent for the one or more therapeutic agents with a solubility of the therapeutic agent(s) in the solvent S1 being 10g/l or higher at 20°C, and a solvent for the one or more polymers, with a solubility of the polymer(s)in the solvent S1 being 100g/l or higher at 20°C, and
(ii) at least one solvent S2 which is fully miscible with solvent S1 and which is partially miscible with water, with a solubility of the solvent S2 in water being 1 to 60 wt% of the solvent S2 in relation to the total weight of a mixed phase containing water and the solvent S2 at 20°C;
b) adding an aqueous surfactant solution with a volume of at least 2 times the volume of the solution provided in step a) to the solution provided in step a) while the solution provided in step a) is stirred, and
c) allowing the nano- and/or microparticles to form via extraction of the solvents S1 and S2 into the aqueous surfactant solution.

2. The process of claim 1, wherein the matrix of the nano- and/or microparticles contains a cellulose ether or cellulose ester selected from methyl cellulose, ethyl cellulose, propyl cellulose, ethyl methyl cellulose, hydroxyethyl cellulose, hydroxypropyl cellulose, hydroxypropyl methyl cellulose, hydroxyethyl methyl cellulose, hydroxyethyl ethyl cellulose, cellulose acetate phthalate (CAP), cellulose acetate, hydroxypropyl methyl cellulose phthalate (HPMCP), hydroxypropyl methylcellulose acetate succinate (HPMC AS) and mixtures thereof.

3. The process of claim 1 or 2, wherein the matrix of the nano- and/or microparticles contains a copolymer of methacrylic acid with one or more (meth)acrylic acid esters selected from poly(methacrylic acid-co-methyl methacrylate), poly(methacrylic acid-co-methyl acrylate), poly(methacrylic acid-co-ethyl acrylate), poly(methacrylic acid-co-ethyl methacrylate), poly(methyl acrylate-co-methyl methacrylate-co-methacrylic acid), and mixtures thereof or a copolymer of two or more (meth)acrylic acid esters selected from poly(ethyl acrylate-co-methyl methacrylate, poly(ethyl acrylate-co-methyl methacrylate-co-trimethylammonioethyl methacrylate chloride), or mixtures thereof.

4. The process of any of claims 1 to 3, wherein the therapeutic agent is selected from raloxifen, atorvastatine, rosuvastatin, quetiapine, duloxetine, asenapine, aripiprazole, methotrexate, glucagon, vasopressin, alendronic acid, clodronic acid, ezetimibe, valsartan, olmesartan, telmisartan, irbesartan, candesartan, bosentan, cytarabine, doxorubicin, irinotecan, diltiazem, verapamil, cortisol, estradiol, progesterone, tamoxifen, morphine, buprenorphine, selegiline, risedronic acid, terbutaline, tiludronic acid, zoledronic acid, ziprasidone, naloxone, pamidronic acid, etidronic acid, albendazole, amidrine, carvedilol, paclitaxel, docetaxel, mesalazine, budesonide, prednisone, paracetamol, dexamethasone, omeprazole, risperidone, L-Dopa, diclofenac, metoprolol, bleomycin, perindopril, trandolapril, ramipril, cilazapril, moexipril, spirapril, fluorouracil, ibuprofen, nifedipine, ondansetron, rivastigmine, simvastatin, losartan, eprosartan, lisinopril and captopril, or, where applicable, from pharmaceutically acceptable salt forms thereof.

5. The process of any of claims 1 to 4, wherein solvent S1 is selected from acetone, dimethyl sulfoxide (DMSO), N-methylpyrrolidone (NMP), glycofurol, tetrahydrofurane, ethanol and mixtures of two or more thereof.

6. The process of any of claims 1 to 5, wherein solvent S2 is selected from alkyl acetates, alkyl formates, dimethyl carbonate, ethyl methyl ketone and mixtures of two or more thereof.

7. The process of any of claims 1 to 6, wherein the concentration of the one or more polymers in the solution provided in step a) is 1 wt% to 40 wt%, based on the total weight of the solution provided in step a).

8. The process of any of claims 1 to 7, wherein the concentration of the surfactant in the aqueous surfactant solution is in the range of 0.1% (w/v) to 30% (w/v), based on the total volume of the surfactant solution, and wherein the surfactant solution has a pH value of 6 or less.

9. The process of any of claims 1 to 8, wherein the aqueous surfactant solution contains a dissolved salt or a dissolved saccharide.

10. The process of any of claims 1 to 9, wherein the nano- and/or microparticles carry a coating formed by a polysaccharide, and wherein the process further comprises the steps of contacting the nano- and/or microparticles formed in step c) with a solution of the polysaccharide, and removing the solvent to form the coating.

11. A pharmaceutical formulation for oral administration comprising nano- and/or microparticles, which particles contain one or more therapeutic agents dispersed in non-crystalline form in a matrix containing one or more polymers selected from cellulose ethers, cellulose esters, copolymers of methacrylic acid with one or more (meth)acrylic acid esters, copolymers of two or more (meth)acrylic acid esters and mixtures thereof, which pharmaceutical formulation is obtainable by the process of any of claims 1 to 10.

12. The pharmaceutical formulation of claim 11, wherein the therapeutic agent is selected from raloxifen, atorvastatine, rosuvastatin, quetiapine, duloxetine, aripiprazole and asenapine, or from pharmaceutically acceptable salt forms thereof.

13. The pharmaceutical formulation of any of claims 11 or 12, wherein the nano- and/or microparticles carry a coating formed from a polysaccharide selected from chitosan, alginate, pectin, inulin, guar gum, dextran, chondroitin sulfate, hyaluronic acid and combinations of two or more thereof.

## Patentansprüche

1. Ein Verfahren zur Herstellung von Nano- und/oder Mikropartikeln zur Verwendung bei oraler Verabreichung,
wobei die Teilchen ein oder mehrere therapeutische Mittel enthalten, die in nicht-kristalliner Form in einer Matrix dispergiert sind, die ein oder mehrere Polymere enthält, ausgewählt aus Celluloseethern, Celluloseestern, Copolymeren von Methacrylsäure mit einem oder mehreren (Meth)acrylsäureestern, Copolymeren von zwei oder mehreren (Meth)acrylsäureestern und Mischungen davon, wobei das Verfahren die folgenden Schritte umfasst
a) Bereitstellung einer Lösung mit
a1) das eine oder die mehreren Therapeutika in gelöster Form;
a2) das eine oder die mehreren Polymere in gelöster Form, und
a3) ein Lösungsmittelgemisch, enthaltend
(i) mindestens ein Lösungsmittel S1, das mit Wasser vollständig mischbar ist und das ein Lösungsmittel für das eine oder die mehreren therapeutischen Mittel ist, wobei die Löslichkeit des/der therapeutischen Mittel(s) in dem Lösungsmittel S1 bei 20°C 10 g/l oder höher ist, und ein Lösungsmittel für das eine oder die mehreren Polymere ist, wobei die Löslichkeit des/der Polymere(s) in dem Lösungsmittel S1 bei 20°C 100 g/l oder höher ist, und
(ii) mindestens einem Lösungsmittel S2, das mit dem Lösungsmittel S1 vollständig mischbar ist und das mit Wasser teilweise mischbar ist, wobei eine Löslichkeit des Lösungsmittels S2 in Wasser 1 bis 60 Gew.-% des Lösungsmittels S2, bezogen auf das Gesamtgewicht einer Wasser und das Lösungsmittel S2 enthaltenden Mischphase bei 20°C, beträgt;
b) Zugabe einer wässrigen Tensidlösung mit einem Volumen von mindestens dem Zweifachen des Volumens der in Schritt a) bereitgestellten Lösung zu der in Schritt a) bereitgestellten Lösung, während die in Schritt a) bereitgestellte Lösung gerührt wird, und
c) Ermöglichung der Bildung der Nano- und/oder Mikropartikel durch Extraktion der Lösungsmittel S1 und S2 in die wässrige Tensidlösung.

2. Verfahren nach Anspruch 1, wobei die Matrix der Nano- und/oder Mikropartikel einen Celluloseether oder Celluloseester enthält, ausgewählt aus Methylcellulose, Ethylcellulose, Propylcellulose, Ethylmethylcellulose, Hydroxyethylcellulose, Hydroxypropylcellulose, Hydroxypropylmethylcellulose, Hydroxyethylmethylcellulose, Hydroxyethylethylcellulose, Celluloseacetatphthalat (CAP), Celluloseacetat, Hydroxypropylmethylcellulosephthalat (HPMCP), Hydroxypropylmethylcelluloseacetatsuccinat (HPMC AS) und Mischungen davon.

3. Verfahren nach Anspruch 1 oder 2, wobei die Matrix der Nano- und/oder Mikropartikel ein Copolymer von Methacrylsäure mit einem oder mehreren (Meth)acrylsäureestern, ausgewählt aus Poly(methacrylsäure-co-methylmethacrylat), Poly(methacrylsäure-co-methylacrylat), Poly(methacrylsäure-co-ethylacrylat), Poly(methacrylsäure-co-ethylmethacrylat), Poly(methacrylsäure-co-ethylmethacrylat), enthält, Poly(methylacrylat-co-methylmethacrylat-co-methacrylsäure) und Mischungen davon oder ein Copolymer aus zwei oder mehreren (Meth)acrylsäureestem, ausgewählt aus Poly(ethylacrylat-co-methylmethacrylat, Poly(ethylacrylat-co-methylmethacrylat-co-trimethylammonioethylmethacrylatchlorid) oder Mischungen davon. se Acetatphthalat (CAP), Celluloseacetat, Hydroxypropylmethylcellulosephthalat (HPMCP), Hydroxypropylmethylcelluloseacetatsuccinat (HPMC AS) und Mischungen davon.

4. Das Verfahren nach einem der Ansprüche 1 bis 3, wobei das therapeutische Mittel ausgewählt ist aus Raloxifen, Atorvastatin, Rosuvastatin, Quetiapin, Duloxetin, Asenapin, Aripiprazol, Methotrexat, Glucagon, Vasopressin, Alendronsäure, Clodronsäure, Ezetimib, Valsartan, Olmesartan, Telmisartan, Irbesartan, Candesartan, Bosentan, Cytarabin, Doxorubicin, Irinotecan, Diltiazem, Verapamil, Cortisol, Estradiol, Progesteron, Tamoxifen, Morphin, Buprenorphin, Selegilin, Risedronsäure, Terbutalin, Tiludronsäure, Zoledronsäure, Ziprasidon, Naloxon, Pamidronsäure, Etidronsäure, Albendazol, Amidrin, Carvedilol, Paclitaxel, Docetaxel, Mesalazin, Budesonid, Prednison, Paracetamol, Dexamethason, Omeprazol, Risperidon, L-Dopa, Diclofenac, Metoprolol, Bleomycin, Perindopril, Trandolapril, Ramipril, Cilazapril, Moexipril, Spirapril, Fluorouracil, Ibuprofen, Nifedipin, Ondansetron, Rivastigmin, Simvastatin, Losartan, Eprosartan, Lisinopril und Captopril oder gegebenenfalls aus pharmazeutisch annehmbaren Salzformen davon.

5. Verfahren nach einem der Ansprüche 1 bis 4, wobei das Lösungsmittel S1 ausgewählt ist aus Aceton, Dimethylsulfoxid (DMSO), N-Methylpyrrolidon (NMP), Glycofurol, Tetrahydrofuran, Ethanol und Mischungen aus zwei oder mehreren davon.

6. Verfahren nach einem der Ansprüche 1 bis 5, wobei das Lösungsmittel S2 ausgewählt ist aus Alkylacetaten, Alkylformiaten, Dimethylcarbonat, Ethylmethylketon und Mischungen aus zwei oder mehreren davon.

7. Verfahren nach einem der Ansprüche 1 bis 6, wobei die Konzentration des einen oder der mehreren Polymere in der in Schritt a) bereitgestellten Lösung 1 Gew.-% bis 40 Gew.-% beträgt, bezogen auf das Gesamtgewicht der in Schritt a) bereitgestellten Lösung.

8. Verfahren nach einem der Ansprüche 1 bis 7, wobei die Konzentration des Tensids in der wässrigen Tensidlösung im Bereich von 0,1 % (Gew./Vol.) bis 30 % (Gew./Vol.) liegt, bezogen auf das Gesamtvolumen der Tensidlösung, und wobei die Tensidlösung einen pH-Wert von 6 oder weniger aufweist.

9. Verfahren nach einem der Ansprüche 1 bis 8, wobei die wässrige Tensidlösung ein gelöstes Salz oder ein gelöstes Saccharid enthält.

10. Verfahren nach einem der Ansprüche 1 bis 9, wobei die Nano- und/oder Mikropartikel eine Beschichtung tragen, die durch ein Polysaccharid gebildet wird, und wobei das Verfahren weiterhin die Schritte des Kontaktierens der in Schritt c) gebildeten Nano- und/oder Mikropartikel mit einer Lösung des Polysaccharids und des Entfernens des Lösungsmittels zur Bildung der Beschichtung umfasst.

11. Pharmazeutische Formulierung zur oralen Verabreichung, umfassend Nano- und/oder Mikroteilchen, wobei die Teilchen ein oder mehrere therapeutische Mittel enthalten, die in nicht-kristalliner Form in einer Matrix dispergiert sind, die ein oder mehrere Polymere enthält, ausgewählt aus Celluloseethern, Celluloseestern, Copolymeren von Methacrylsäure mit einem oder mehreren (Meth)acrylsäureestem, Copolymeren von zwei oder mehreren (Meth)acrylsäureestem und Mischungen davon, wobei die pharmazeutische Formulierung durch das Verfahren nach einem der Ansprüche 1 bis 10 erhältlich ist.

12. Pharmazeutische Formulierung nach Anspruch 11, wobei das therapeutische Mittel ausgewählt ist aus Raloxifen, Atorvastatin, Rosuvastatin, Quetiapin, Duloxetin, Aripiprazol und Asenapin oder aus pharmazeutisch annehmbaren Salzformen davon.

13. Pharmazeutische Formulierung nach einem der Ansprüche 11 oder 12, wobei die Nano-und/oder Mikropartikel eine Beschichtung tragen, die aus einem Polysaccharid gebildet ist, das aus Chitosan, Alginat, Pektin, Inulin, Guargummi, Dextran, Chondroitinsulfat, Hyaluronsäure und Kombinationen von zwei oder mehreren davon ausgewählt ist.

## Revendications

1. Un procédé de production de nano- et/ou microparticules pour l'administration orale, lesquelles particules contiennent un ou plusieurs agents thérapeutiques dispersés sous forme non cristalline dans une matrice contenant un ou plusieurs polymères choisis parmi les éthers de cellulose, les esters de cellulose, les copolymères d'acide méthacrylique avec un ou plusieurs esters d'acide (méth)acrylique, les copolymères de deux ou plusieurs esters d'acide (méth)acrylique et leurs mélanges, ledit procédé comprenant les étapes suivantes
a) fournir une solution contenant
a1) le ou les agents thérapeutiques sous forme dissoute ;
a2) un ou plusieurs polymères sous forme dissoute, et
a3) un mélange de solvants contenant
(i) au moins un solvant S1 qui est totalement miscible à l'eau et qui est un solvant pour un ou plusieurs agents thérapeutiques, la solubilité du ou des agents thérapeutiques dans le solvant S1 étant de 10 g/l ou plus à 20°C, et un solvant pour un ou plusieurs polymères, la solubilité du ou des polymères dans le solvant S1 étant de 100 g/l ou plus à 20°C, et
(ii) au moins un solvant S2 qui est totalement miscible avec le solvant S1 et qui est partiellement miscible avec l'eau, la solubilité du solvant S2 dans l'eau étant de 1 à 60 % en poids du solvant S2 par rapport au poids total d'une phase mixte contenant de l'eau et le solvant S2 à 20°C ;
b) l'addition d'une solution aqueuse d'agent de surface d'un volume au moins égal à deux fois le volume de la solution prévue à l'étape a) à la solution prévue à l'étape a) tout en agitant la solution prévue à l'étape a), et
c) laisser les nano- et/ou microparticules se former par extraction des solvants S1 et S2 dans la solution aqueuse de tensioactif.

2. Procédé de la revendication 1, dans lequel la matrice des nano- et/ou microparticules contient un éther de cellulose ou un ester de cellulose choisi parmi la méthylcellulose, l'éthylcellulose, la propylcellulose, l'éthylméthylcellulose, l'hydroxyéthylcellulose, l'hydroxypropylcellulose, hydroxypropyl méthyl cellulose, hydroxyéthyl méthyl cellulose, hydroxyéthyl éthyl cellulose, acétate phtalate de cellulose (CAP), acétate de cellulose, phtalate d'hydroxypropyl méthyl cellulose (HPMCP), acétate succinate d'hydroxypropyl méthylcellulose (HPMC AS) et leurs mélanges.

3. Procédé selon la revendication 1 ou 2, dans lequel la matrice des nano- et/ou microparticules contient un copolymère d'acide méthacrylique avec un ou plusieurs esters d'acide (méth)acrylique choisis parmi le poly(acide méthacrylique-co-méthacrylate de méthyle), le poly(acide méthacrylique-co-acrylate de méthyle), le poly(acide méthacrylique-co-acrylate d'éthyle), le poly(acide méthacrylique-co-méthacrylate d'éthyle), poly(acrylate de méthyle-co-méthacrylate de méthyle-co-acide méthacrylique), et leurs mélanges ou un copolymère de deux ou plusieurs esters d'acide (méth)acrylique choisis parmi le poly(acrylate d'éthyle-co-méthacrylate de méthyle, le poly(acrylate d'éthyle-co-méthacrylate de méthyle-co-chlorure de méthacrylate de triméthylammonioéthyle), ou leurs mélanges. se acétate phtalate (CAP), acétate de cellulose, phtalate d'hydroxypropylméthylcellulose (HPMCP), acétate succinate d'hydroxypropylméthylcellulose (HPMC AS) et leurs mélanges.

4. Procédé pour l'une quelconque des revendications 1 à 3, dans lequel l'agent thérapeutique est choisi parmi le raloxifène, l'atorvastatine, la rosuvastatine, la quétiapine, la duloxétine, l'asénapine, l'aripiprazole, le méthotrexate, le glucagon, la vasopressine, l'acide alendronique, l'acide clodronique, l'ézétimibe, le valsartan, olmésartan, telmisartan, irbesartan, candésartan, bosentan, cytarabine, doxorubicine, irinotécan, diltiazem, vérapamil, cortisol, estradiol, progestérone, tamoxifène, morphine, buprénorphine, sélégiline, acide risédronique, terbutaline, acide tiludronique, acide zolédronique, ziprasidone, naloxone, acide pamidronique, acide étidronique, albendazole, amidrine, carvédilol, paclitaxel, docétaxel, mésalazine, budésonide, prednisone, paracétamol, dexaméthasone, oméprazole, rispéridone, L-Dopa, diclofénac, métoprolol, bléomycine, périndopril, trandolapril, ramipril, cilazapril, moexipril, spirapril, fluorouracil, ibuprofène, nifédipine, ondansétron, rivastigmine, simvastatine, losartan, éprosartan, lisinopril et captopril, ou, le cas échéant, à partir de leurs formes salines pharmaceutiquement acceptables.

5. Procédé selon l'une des revendications 1 à 4, dans lequel le solvant S1 est choisi parmi l'acétone, le diméthyl sulfoxyde (DMSO), la N-méthylpyrrolidone (NMP), le glycofurol, le tétrahydrofurane, l'éthanol et les mélanges de deux ou plusieurs de ces substances.

6. Procédé pour l'une quelconque des revendications 1 à 5, dans lequel le solvant S2 est choisi parmi les acétates d'alkyle, les formiates d'alkyle, le carbonate de diméthyle, l'éthylméthylcétone et les mélanges de deux ou plusieurs d'entre eux.

7. Procédé selon l'une quelconque des revendications 1 à 6, dans lequel la concentration d'un ou plusieurs polymères dans la solution fournie à l'étape a) est de 1 % en poids à 40 % en poids, sur la base du poids total de la solution fournie à l'étape a).

8. Procédé selon l'une des revendications 1 à 7, dans lequel la concentration de l'agent de surface dans la solution aqueuse d'agent de surface est comprise entre 0,1 % (p/v) et 30 % (p/v), sur la base du volume total de la solution d'agent de surface, et dans lequel la solution d'agent de surface a une valeur de pH de 6 ou moins.

9. Procédé selon l'une des revendications 1 à 8, dans lequel la solution aqueuse d'agent de surface contient un sel dissous ou un saccharide dissous.

10. Procédé selon l'une quelconque des revendications 1 à 9, dans lequel les nanoparticules et/ou microparticules portent un revêtement formé par un polysaccharide, et dans lequel le procédé comprend en outre les étapes consistant à mettre en contact les nanoparticules et/ou microparticules formées à l'étape c) avec une solution du polysaccharide, et à éliminer le solvant pour former le revêtement.

11. Formulation pharmaceutique pour administration orale comprenant des nano- et/ou microparticules, lesquelles particules contiennent un ou plusieurs agents thérapeutiques dispersés sous forme non cristalline dans une matrice contenant un ou plusieurs polymères choisis parmi les éthers de cellulose, les esters de cellulose, les copolymères d'acide méthacrylique avec un ou plusieurs esters d'acide (méth)acrylique, les copolymères de deux ou plusieurs esters d'acide (méth)acrylique et leurs mélanges, laquelle formulation pharmaceutique peut être obtenue par le procédé de l'une des revendications 1 à 10.

12. Formulation pharmaceutique selon la revendication 11, dans laquelle l'agent thérapeutique est choisi parmi le raloxifène, l'atorvastatine, la rosuvastatine, la quétiapine, la duloxétine, l'aripiprazole et l'asénapine, ou parmi leurs formes salines pharmaceutiquement acceptables.

13. Formulation pharmaceutique selon l'une quelconque des revendications 11 ou 12, dans laquelle les nano- et/ou microparticules portent un enrobage formé d'un polysaccharide choisi parmi le chitosane, l'alginate, la pectine, l'inuline, la gomme de guar, le dextrane, le sulfate de chondroïtine, l'acide hyaluronique et des combinaisons de deux ou plusieurs de ceux-ci.
